# EUROPÄISCHE PATENTSCHRIFT

(11) **EP 0 669 968 B1**
(45) Veröffentlichungstag und Bekanntmachung des Hinweises auf die Patenterteilung: **05.02.1997**
(21) Anmeldenummer: 94900118.4
(22) Anmeldetag: 09.11.1993
(51) Int. Cl.: C10L 1/00, C10M 171/00, C10L 1/22

(54) **ANILINE ALS MARKIERUNGSMITTEL FÜR MINERALÖLE**
ANILINES AS MARKING AGENTS FOR MINERAL OILS
ANILINES UTILISEES COMME AGENTS DE MARQUAGE POUR DES HUILES MINERALES

(30) Priorität: 19.11.1992 DE 4238994
(43) Veröffentlichungstag der Anmeldung: 06.09.1995
(73) Patentinhaber: BASF Aktiengesellschaft, 67063 Ludwigshafen (DE)
(72) Erfinder: DYLLICK-BRENZINGER, Rainer, D-6940 Weinheim (DE); RAULFS, Friedrich-Wilhelm, D-6700 Ludwigshafen (DE); SCHLOESSER, Ulrike, D-6800 Mannheim 81 (DE)
(86) Internationale Anmeldenummer: EP9303133
(87) Internationale Veröffentlichungsnummer: WO9411466

(56) Entgegenhaltungen:
- EP-A- 0 499 845
- US-A- 2 689 171
- US-A- 4 209 302

## Beschreibung

Die vorliegende Erfindung betrifft die Verwendung von Anilinen der Formel I 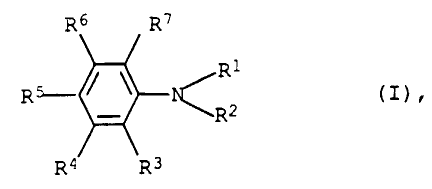 in der
- R¹ und R²: unabhängig voneinander jeweils Wasserstoff, C₁-C₁₈-Alkyl, das gegebenenfalls substituiert ist und durch 1 bis 3 Sauerstoffatome in Etherfunktion oder 1 bis 3 C₁-C₄-Alkyliminogruppen unterbrochen sein kann, C₃-C₁₈-Alkenyl, gegebenenfalls substituiertes Phenyl oder R¹ und R² zusammen mit dem sie verbindenden Stickstoffatom einen 5- oder 6-gliedrigen gesättigten heterocyclischen Rest, der ein weiteres Heteroatom aufweisen kann, oder R¹ auch einen Rest der Formel OL¹ oder NL¹L², worin L¹ und L² unabhängig voneinander jeweils für Wasserstoff, gegebenenfalls substituiertes C₁-C₁₈-Alkyl, C₃-C₁₈-Alkenyl oder gegebenenfalls substituiertes Phenyl stehen,
- R³ und R⁷: unabhängig voneinander jeweils Wasserstoff, gegebenenfalls substituiertes C₁-C₁₈-Alkyl, C₂-₁₈-Alkenyl, Cyano, Nitro, Formyl, gegebenenfalls substituiertes C₂-C₄-Alkanoyl, Formylamino, gegebenenfalls substituiertes C₂-C₄-Alkanoylamino, Benzoylamino oder einen Rest der Formel OL¹, CH₂COOL¹, NL¹L², SL¹ oder SO₂NL¹L², worin L¹ und L² jeweils die obengenannte Bedeutung besitzen, oder zusammen mit R² C₂-C₃-Alkylen, das gegebenenfalls ein bis dreifach durch Methyl substituiert ist, oder gegebenenfalls durch Phenyl substituiertes C₂-C₃-Alkenylen,
- R⁴: Wasserstoff, gegebenenfalls substituiertes C₁-C₁₈-Alkyl, C₂-C₁₈-Alkenyl, Nitro oder einen Rest der Formel OL¹, NL¹L², COOL¹ oder SO₂NL¹L², worin L¹ und L² jeweils die obengenannte Bedeutung besitzen, und
- R⁵ und R⁶: unabhängig voneinander jeweils Wasserstoff, gegebenenfalls substituiertes C₁-C₁₈-Alkyl, C₂-C₁₈-Alkenyl, gegebenenfalls substituiertes Phenyl, Nitro, Formylamino, gegebenenfalls substituiertes C₂-C₄-Alkanoylamino, Benzoylamino oder einen Rest der Formel OL¹, NL¹L², SO₂NL¹L², SO₂L³, COL¹ oder COOL¹, worin L¹ und L² jeweils die obengenannte Bedeutung besitzen und L³ für gegebenenfalls substituiertes C₁-C₁₈-Alkyl, C₃-C₁₈-Alkenyl oder gegebenenfalls substituiert Phenyl steht, bedeuten,
mit der Maßgabe, daß mindestens einer der Reste R³, R⁵ oder R⁷ Wasserstoff bedeutet,
zum Markieren von Mineralölen, wobei die Aniline der Formel I bei ihrem Nachweis als Markierungsstoff mit einem Diazoniumsalz unter Bildung eines Azofarbstoffs gekuppelt werden.

Aus der US-A-4 209 302 ist die Verwendung von α- oder β-Naphthylamin, das an der Aminogruppe einen durch eine weitere Aminogruppe substituierten Propylrest trägt, zum Markieren von Mineralölen bekannt. Der Nachweis des Markers wird dabei durch Umsetzung des Naphthylamins mit diazotiertem 2-Chlor-4-nitroanilin vorgenommen. Es hat sich jedoch gezeigt, daß diese Markierungs- und Nachweismethode noch nicht voll befriedigend ist.

Weiterhin beschreibt die US-A-2 689 171 die Markierung von Mineralölen mit Anilin oder p-Toluidin, wobei diese Produkte als Diazokomponenten für die Bildung von Azofarbstoffen dienen, d.h. bei ihrem Nachweis werden sie diazotiert und mit einer Kupplungskomponente, z.B. β-Naphthol, gekuppelt.

Aufgabe der vorliegenden Erfindung war es nun, neue Mittel zum Markieren von Mineralölen bereitzustellen. Die neuen Mittel sollten leicht zugänglich und gut in Mineralölen löslich sein. Außerdem sollten sie in einfacher Weise nachgewiesen werden können. Dabei sollten selbst noch sehr kleine Mengen an Markierstoff durch eine starke Farbreaktion sichtbar gemacht werden können. Schließlich sollte der Marker nicht durch einfache Extraktion mit Wasser aus dem markiertem Mineralöl entfernbar sein.

Demgemäß wurde gefunden, daß sich die eingangs näher bezeichneten Aniline der Formel I vorteilhaft zum Markieren von Mineralölen eignen.

Alle in der obengenannten Formel I auftretenden Alkyl- und Alkenylreste können sowohl geradkettig als auch verzweigt sein.

Im erfindungsgemäßen Sinn sind unter Alkenylresten im wesentlichen solche Reste zu verstehen, die 1 bis 3 Doppelbindungen aufweisen.

Wenn in der obengenannten Formel I substituierte Alkylgruppen auftreten, so können als Substituenten z.B. Hydroxy, C₁-C₄-Alkoxy, Phenoxy, Cyano, Phenyl, C₁-C₄-Dialkylamino, C₁-C₄-Alkanoyloxy, 1-(C₁-C₄-Alkoxy)-C₂-C₄-alkoxy, N-(C₁-C₄-Alkyl)-N-(hydroxy-C₂-C₄-alkyl)carbamoyl, C₁-C₄-Alkoxycarbonyl, Phenoxycarbonyloxy, C₁-C₄-Alkylaminocarbonyloxy, Phenylaminocarbonyloxy oder Acetacetyloxy in Betracht kommen. Die Alkylgruppen weisen dabei in der Regel 1 oder 2 Substituenten auf.

Wenn in der obengenannten Formel I substituierte Phenylgruppen auftreten, so können als Substituenten z.B. C₁-C₄-Alkyl oder C₁-C₄-Alkoxy in Betracht kommen. Die Phenylgruppen weisen dabei in der Regel 1 bis 3 Substituenten auf.

Wenn R¹ und R² zusammen mit dem sie verbindenden Stickstoffatom einen 5- oder 6-gliedrigen gesättigten heterocyclischen Rest, der ein weiteres Heteroatom aufweisen kann, bedeuten, so können dafür z.B. Pyrrolidinyl, Piperidinyl, Morpholinyl, Piperazinyl oder N-(C₁-C₄-Alkyl)piperazinyl in Betracht kommen.

Reste R¹, R², R³, R⁴, R⁵, R⁶, R⁷, L¹, L² und L³ sind z.B. Methyl, Ethyl, Propyl, Isopropyl, Butyl, Isobutyl, sec-Butyl, Pentyl, Isopentyl, Neopentyl, tert-Pentyl, Hexyl, 2-Methylpentyl, Heptyl, Octyl, 2-Ethylhexyl, Isooctyl, Nonyl, Isononyl, Decyl, Isodecyl, Undecyl, Dodecyl, Tridecyl, 3,5,5,7-Tetramethylnonyl, Isotridecyl (die obigen Bezeichnungen Isooctyl, Isononyl, Isodecyl und Isotridecyl sind Trivialbezeichnungen und stammen von den nach der Oxosynthese erhaltenen Alkoholen - vgl. dazu Ullmanns Encyklopädie der technischen Chemie, 4. Auflage, Band 7, Seiten 215 bis 217, sowie Band 11, Seiten 435 und 436), Tetradecyl, Pentadecyl, Hexadecyl, Heptadecyl, Octadecyl, 2-Methoxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 2- oder 3-Propoxypropyl, 2- oder 3-Butoxypropyl, 2- oder 4-Methoxybutyl, 2- oder 4-Ethoxybutyl, 2- oder 4-Propoxybutyl, 2- oder 4-Butoxybutyl, Cyanomethyl, 2-Cyanoethyl, 3-Cyanopropyl, 2-Cyanobutyl, 4-Cyanobutyl, 5-Cyanopentyl, 6-Cyanohexyl, 2-Hydroxyethyl, 2-Hydroxypropyl, 3-Hydroxypropyl, 2-Hydroxybutyl, 4-Hydroxybutyl, 5-Hydroxypentyl, 6-Hydroxyhexyl, 5-Hydroxy-3-oxapentyl, Benzyl, 1-Phenylethyl, 2-Phenylethyl, 2-Formyloxyethyl, 2-Acetyloxyethyl, 2-Propionyloxyethyl, 2-Isobutyryloxyethyl, 2- oder 3-Formyloxypropyl, 2- oder 3-Acetyloxypropyl, 2- oder 3-Propionyloxypropyl, 2- oder 3-Isobutyryloxypropyl, 2- oder 4-Formyloxybutyl, 2- oder 4-Acetyloxybutyl, 2- oder 4-Propionyloxybutyl, 2- oder 4-Isobutyryloxybutyl, N-Methyl-N-(2-hydroxyethyl)-carbamoyl, 2-Methoxycarbonylethyl, 2-Ethoxycarbonylethyl, 2-Phenoxycarbonyloxyethyl, 2-Methylaminocarbonyloxyethyl, 2-Ethylaminocarbonyloxyethyl, 2-Isopropylaminocarbonyloxyethyl, 2-Phenylaminocarbonyloxyethyl, 2-Acetacetyloxyethyl, Allyl, prop-1-en-1-yl, Methallyl, Ethallyl, Pentenyl, Pentadienyl, Hexadienyl, 3,7-Dimethylocta-1,6-dien-1-yl, Undec-10-en-1-yl, 6,10-Dimethylundeca-5,9-dien-2-yl, 3,7-11-Trimethyldodeca-1,6,10-trien-1-yl, 3,7,11-Trimethyldodeca-2,6, 10-trien-1-yl, Octadec-9-en-1-yl, Octadeca-9,12-dien-1-yl, Octadeca-9,12,15-trien-1-yl, 6,10,14-Trimethylpentadeca-5,9, 13-trien-2-yl, Hydroxy, Methoxy, Ethoxy, Propoxy, Isopropoxy, Butoxy, Isobutoxy, sec-Butoxy, Pentyloxy, Isopentyloxy, Neopentyloxy, tert-Pentyloxy, Hexyloxy, Heptyloxy, Octyloxy, Isooctyloxy, 2-Ethylhexyloxy, Nonlyoxy, Isononyloxy, Decyloxy, Isodecyloxy, Undecyloxy, Dodecyloxy, Tridecyloxy, Isotridecyloxy, Tetradecyloxy, Pentadecyloxy, Hexadecyloxy, Heptadecyloxy, Octadecyloxy, Amino, Mono- oder Dimethylamino, Mono- oder Diethylamino, Mono- oder Dipropylamino, Mono- oder Diisopropylamino, Mono- oder Dibutylamino, N-Methyl-N-ethylamino, Mono- oder Diallylamino, Phenylamino oder N-Phenyl-N-methylamino.

Reste R¹ und R² sind weiterhin z.B. 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 4,8-Dioxadecyl, 3,6,8-Trioxadecyl, 3,6,9-Trioxaundecyl, 2-Dimethylaminoethyl, 2-Diethylaminoethyl, 2- oder 3-Dimethylaminopropyl, 2- oder 3-Diethylaminopropyl, 2- oder 4-Dimethylaminopropyl, 2- oder 4-Diethylaminobutyl, 3,6-Dimethyl-3,6-diazaheptyl, 3,6,9-Trimethyl-3,6,9-triazadecyl, 2-(1-Methoxyethoxy)ethyl, 2-(1-Ethoxyethoxy)ethyl, 2-(1-Isobutoxyethoxy)ethyl, 2- oder 3-(1-Methoxyethoxy)propyl, 2- oder 3-(1-Ethoxyethoxy)propyl oder 2- oder 3-(1-Isobutoxyethoxy)propyl.

Reste R¹, R², R⁵ und R⁶ sind weiterhin z.B. Phenyl, 2-, 3- oder 4-Methylphenyl, 2-, 3- oder 4-Ethylphenyl, 2,4-Dimethylphenyl, 2-, 3- oder 4-Methoxyphenyl, 2-, 3- oder 4-Ethoxyphenyl oder 2,4-Dimethoxyphenyl.

Reste R³, R⁵, R⁶ und R⁷ sind weiterhin z.B. Formylamino, Acetylamino, Propionylamino, Butyrylamino oder Isobutyrylamino.

Reste R³, R⁴, R⁵, R⁶ und R⁷ sind weiterhin z.B. Vinyl, Sulfamoyl, Mono- oder Dimethylsulfamoyl, Mono- oder Diethylsulfamoyl, Mono- oder Dipropylsulfamoyl, Mono- oder Diisopropylsulfamoyl, Mono- oder Dibutylsulfamoyl, N-Methyl-N-ethylsulfamoyl, Mono- oder Diallylsulfamoyl oder Phenylsulfamoyl.

Reste R³ und R⁷ sind weiterhin z.B. Carboxylmethyl, Methoxycarbonylmethyl, Ethoxycarbonylmethyl, Propoxycarbonylmethyl, Isopropoxycarbonylmethyl, Butoxycarbonylmethyl, Allyloxycarbonylmethyl, Phenoxycarbonylmethyl, Mercapto, Methylthio, Ethylthio, Propylthio, Isopropylthio oder Butylthio.

Reste R⁴, R⁵ und R⁶ sind weiterhin z.B. Carboxyl, Methoxycarbonyl, Ethoxycarbonyl, Propoxycarbonyl, Isopropoxycarbonyl, Butoxycarbonyl, Allyloxycarbonyl, Phenoxycarbonyl, Allylthio oder Phenylthio.

Reste R⁵ und R⁶ sind weiterhin z.B. Methylsulfonyl, Ethylsulfonyl, Propylsulfonyl, Isopropylsulfonyl, Butylsulfonyl, Phenylsulfonyl, Formyl, Acetyl, Propionyl, Butyryl, Isobutyryl oder Benzoyl.

Reste R² und R³ oder R² und R⁷ zusammen sind z.B. 1,3-Propylen, 1,1,3-Trimethyl-1,3-propylen, Vinylen, Phenylvinylen oder ein Rest der Formel CH=CH-CH₂.

Bevorzugt werden Aniline der Formel I, in der
- R¹ und R²: unabhängig voneinander jeweils Wasserstoff, C₁-C₁₅-Alkyl, das gegebenenfalls substituiert ist und durch 1 bis 3 Sauerstoffatome in Etherfunktion oder 1 bis 3 C₁-C₄-Alkyliminogruppen unterbrochen sein kann, Allyl, gegebenenfalls substituiertes Phenyl oder R¹ und R² zusammen mit dem sie verbindenden Stickstoffatom einen 5- oder 6-gliedrigen gesättigten heterocyclischen Rest, der ein weiteres Heteroatom aufweisen kann,
- R³ und R⁷: unabhänig voneinander jeweils Wasserstoff, C₁-C₄-Alkoxy oder zusammen mit R² 1,3-Propylen, das gegebenenfalls ein- bis dreifach durch Methyl substituiert ist,
- R⁴ und R⁵: jeweils Wasserstoff und
- R⁶: Wasserstoff, C₁-C₄-Alkyl, C₁-C₄-Alkoxy, C₁-C₄-Alkanoylamino oder Benzoylamino bedeuten,
zum Markieren von Mineralölen verwendet.

Besonders bevorzugt werden Aniline der Formel I, in der R¹ und R² jeweils Wasserstoff oder C₁-C₄-Alkyl oder mindestens einer der beiden Reste R¹ und R² C₂-C₁₅-Alkyl, das durch Hydroxy substituiert ist und durch 1 bis 3 Sauerstoffatome in Etherfunktion oder 1 bis 3 C₁-C₄-Alkyliminogruppen unterbrochen sein kann, und R⁶ C₁-C₄-Alkyl, insbesondere Methyl, bedeuten, zum Markieren von Mineralölen verwendet.

Besonders bevorzugt werden weiterhin Aniline der Formel I, in der R³ Wasserstoff, C₁-C₄-Alkyl oder C₁-C₄-Alkoxy und R⁶ Wasserstoff, C₁-C₄-Alkyl, C₁-C₄-Alkoxy, Amino oder Acetylamino bedeuten, zum Markieren von Mineralölen verwendet.

Besonders bevorzugt werden weiterhin Aniline der Formel I, in der R⁷ Wasserstoff, C₁-C₄-Alkyl oder C₁-C₄-Alkoxy bedeutet, zum Markieren von Mineralölen verwendet.

Insbesondere verwendet man Aniline der Formel I, in der mindestens einer der beiden Reste R¹ und R² C₂-C₄-Alkyl, das durch Hydroxy substituiert ist, und R⁶ Methyl bedeuten, zum Markieren von Mineralölen.

Hervorzuheben ist die Verwendung von Anilinen der Formel I, in der R¹ und R² jeweils C₂-C₄-Alkyl, das durch Hydroxy substituiert ist, insbesondere 2-Hydroxyethyl oder 2-oder 3-Hydroxypropyl oder R¹ C₂-C₄-Alkyl, das durch Hydroxy substituiert ist, insbesondere 2-Hydroxyethyl oder 2- oder 3-Hydroxypropyl und R² C₁-C₄-Alkyl bedeuten, zum Markieren von Mineralölen.

Hervorzuheben ist weiterhin die Verwendung von 2-Methoxy- oder 2-Ethoxy-5-acetylaminoanilin zum Markieren von Mineralölen.

Ein weiterer Gegenstand der vorliegenden Erfindung sind Mineralöle, enthaltend eines oder mehrere der Aniline der Formel I.

Unter Mineralölen im erfindungsgemäßen Sinn sind beispielsweise Treibstoffe, wie Benzin, Kerosin oder Dieselöl, oder Öle, wie Heizöl oder Motorenöl, zu verstehen.

Die Aniline der Formel I eignen sich insbesondere zum Markieren von Mineralölen, bei denen eine Kennzeichnung gefordert wird, z.B. aus steuerlichen Gründen. Um die Kosten der Kennzeichnung gering zu halten, strebt man dabei an, für die Markierung möglichst geringe Mengen an Markierungsmittel anzuwenden.

Zum Markieren von Mineralöl werden die Aniline der Formel I entweder in Substanz oder in Form von Lösungen angewandt. Als Lösungsmittel eignen sich vorzugsweise aromatische Kohlenwasserstoffe, wie Dodecylbenzol, Diisopropylnaphthalin oder ein Gemisch höherer Aromaten, das unter dem Namen Shellsol® AB (Fa. Shell) handelsüblich ist. Um eine hohe Viskosität der resultierenden Lösungen zu vermeiden, wählt man im allgemeinen eine Konzentration an Anilin I von 30 bis 50 Gew.-%, bezogen auf die Lösung.

Mittels den erfindungsgemäß anzuwendenden Anilinen der Formel I gelingt es sehr einfach, markierte Mineralöle nachzuweisen, selbst wenn die Markierungssubstanzen nur in einer Konzentration von ungefähr 10 ppm oder darunter vorliegen.

Der Nachweis der als Markierungsstoffe angewandten Aniline der Formel I in Mineralölen gelingt vorteilhaft, wenn man das Anilin der Formel I durch Behandeln des Mineralöls mittels eines wäßrigen Mediums extrahiert und in der wäßrigen Phase, gegebenenfalls in Gegenwart eines Puffers, mit einem Diazoniumsalz, das sich von einem Amin aus der Aminoanthrachinon-, Aminonaphthalin-, Anilin-, Aminothiophen-, Aminothiazol- oder Aminobenzisothiazolreihe ableitet, unter Bildung eines Azofarbstoffs kuppelt.

Der Nachweis der als Markierungsstoffe angewandten Aniline der Formel I in Mineralölen gelingt besonders vorteilhaft, wenn man das Anilin der Formel I durch Behandeln des Mineralöls mittels einer wäßrigen Lösung des Diazoniumsalzes extrahiert und anschließend, gegebenenfalls in Gegenwart eines Puffers, mit dem Diazoniumsalz unter Bildung eines Azofarbstoffs kuppelt.

Geeignete Säuren, die den sauren wäßrigen Lösungen zugrundeliegen, sind z.B. anorganische oder organische Säuren, wie Salzsäure, Schwefelsäure, Salpetersäure, Phosphorsäure, Ameisensäure, Essigsäure oder Propionsäure. Die sauren wäßrigen Lösungen weisen im allgemeinen eine Säurekonzentration von 0,5 bis 20 Gew.-% auf.

Der Nachweis der als Markierungsstoffe angewandten Aniline der Formel I in Mineralölen gelingt weiterhin besonders vorteilhaft, wenn man das Anilin der Formel I durch Behandeln des Mineralöls mittels wäßrigen Mediums extrahiert und in der wäßrigen Phase, gegebenenfalls in Gegenwart eines Puffers, mit dem Diazoniumsalz, das sich in festem Aggregatzustand auf einem Träger befindet, unter Bildung eines Azofarbstoffs kuppelt.

Geeignete wäßrige Medien zur Extraktion des Anilins der Formel I aus dem Mineralöl sind z.B. Wasser oder Gemische von Wasser mit Säuren und/oder wassermischbaren organischen Lösungsmitteln und/oder anorganischen Substanzen.

Als Säuren kommen dabei die obengenannten Säuren in der angegebenen Konzentration in Betracht.

Wassermischbare organische Lösungsmittel sind z.B. Alkohole, wie Methanol, Ethanol, Propanol, Isopropanol, Ethylglykol oder 1,2-Propylenglykol, Ether, wie 2-Methoxyethanol, 2-Ethoxyethanol, 2-Butoxyethanol, 2-(2-Methoxyethoxy)ethanol, 1-Methoxypropan-2-ol oder Tetrahydrofuran, Carbonsäureamide, wie N,N-Dimethylformamid oder N-Methylpyrrolidinon, Propylencarbonat, Dimethylsulfoxid oder Sulfolan. Der Anteil an wassermischbarem organischem Lösungsmittel liegt im allgemeinen bei 1 bis 50 Gew.-%, bezogen auf das Gewicht des wäßrigen Mediums.

Anorganische Substanzen sind z.B. Salze, wie Alkalihalogenide, Aluminiumhalogenide oder Zinkhalogenide, oder Ammoniak.

Die Anwendung von wäßriger Säure, die gegebenenfalls noch ein wassermischbares organisches Lösungsmittel enthält, als wäßriges Medium ist bevorzugt.

Geeignete Träger sind anorganische Materialien, wie Aktivkohle, Molekularsiebe, Kieselgur, Titandioxid, Aluminiumoxid oder Calciumchlorid, oder organische Materialien, wie Cellulosefasern, Baumwolle, Holzschliff, Polystyrol oder Polyvinylchlorid.

Nach der Zugabe des auf dem Träger befindlichen Diazoniumsalzes zum wäßrigen Extrakt des Anilins der Formel I wird das Diazoniumsalz ganz oder teilweise gelöst und so für die Azokupplung zur Verfügung gestellt.

Um zu einer optimalen Kupplungsreaktion, die gegebenenfalls in Gegenwart eines Lösungsmittels verläuft, und daraus resultierend zu einer Optimierung der Azofarbstoffausbeute zu gelangen, empfiehlt es sich, den pH-Wert durch Anwendung von Puffersubstanzen zu kontrollieren und die Reaktionspartner im günstigen Molverhältnis (Anilin : Diazoniumsalz 1 : 500 bis 1 : 1, vorzugsweise 1 : 100 bis 1 : 20) anzuwenden.

Geeignete Puffersubstanzen sind z.B. Alkaliacetate, Monoalkalicitrate, Alkalidihydrogenphosphate, wobei insbesondere jeweils die Natriumsalze zu nennen sind, oder solche Puffersysteme, wie sie im Handbook of Chemistry and Physics, 65th, Ed., 1984-1985, Seiten D148 bis 150, genannt sind.

Als vorteilhaft hat sich der Nachweis der Aniline der Formel I mittels eines "Diazopapiers" oder einer "Diazofolie" erwiesen. Diese Diazoindikatoren werden durch Tränken eines Papiers, z.B. eines Filterpapiers, oder einer Dünnschichtchromatographiefolie, die z.B. mit Cellulose beschichtet ist, mit der entsprechenden Diazoniumsalzlösung hergestellt, wobei man den Bereich unbehandelt läßt, der beim Entwickeln direkt in Kontakt mit dem wäßrigen Extrakt kommt. Durch Eintauchen eines so hergestellten Papier- oder Folienstreifens in den wäßrigen Extrakt läßt sich die Azofarbstoffausbeute nach Art einer Papierchromatographie optimieren. Der dabei sich bildende Azofarbstoff bleibt am Start und wird durch laufend nachgeliefertes Anilin aufkonzentriert und ist deshalb gut nachzuweisen.

Als Amin aus der Anthrachinonreihe sind 1-Aminoanthrachinon und 4-Chlor-1-aminoanthrachinon besonders hervorzuheben.

Geeignete Aminonaphthaline gehorchen z.B. der Formel II 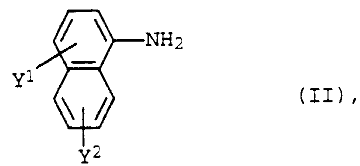 in der Y¹ und Y² unabhängig voneinander jeweils Wasserstoff, Hydroxy oder Hydroxysulfonyl bedeuten.

Geeignete Aniline, die den Diazoniumsalzen zugrundeliegen, gehorchen z.B. der Formel III 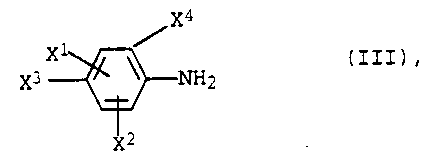 in der
- X¹: Wasserstoff, Halogen, C₁-C₄-Alkoxy, Nitro oder Hydroxysulfonyl,
- X²: Wasserstoff, Halogen, C₁-C₄-Alkoxy, Cyano, Nitro, oder gegebenenfalls durch Methyl, Ethyl, Methoxy oder Ethoxy substituiertes Phenylazo,
- X³: Wasserstoff, C₁-C₄-Dialkylamino, Pyrrolidino, Piperidino, Morpholino, Nitro oder Hydroxysulfonyl und
- X⁴: Wasserstoff, Halogen, Cyano oder einen heterocyclischen Rest, z.B. 3-Phenyl-1,2,4-oxdiazol-5-yl, bedeuten.

Geeignete Aminothiophene, Aminothiazole oder Aminobenzisothiazole gehorchen z.B. der Formel

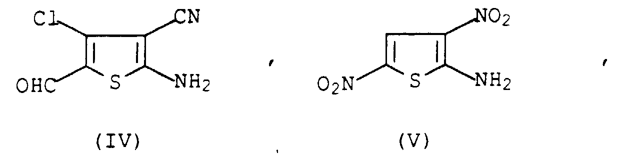

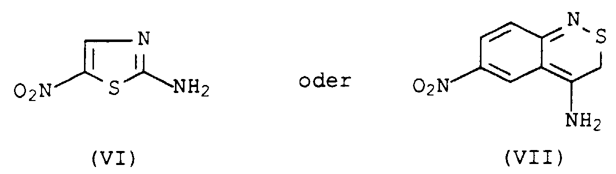

Geeignete Anionen, die als Gegenionen zu den Diazoniumkationen in Betracht kommen, sind die dort üblichen Anionen, wie Chlorid, Bromid, Hydrogensulfat, Sulfat, Dihydrogenphosphat, Monohydrogenphosphat, Phosphat, Tetrafluoroborat, Tetrachlorozinkat, Naphthalin-1,5-disulfonat oder Acetat.

In manchen Fällen ist es vorteilhaft, noch geringe Mengen an Alkalisalzen von Arylsulfonsäuren, z.B. das Natriumsalz der Naphthalin-1,5-disulfonsäure, als Stabilisator für die Diazoniumsalze zuzusetzen.

Bevorzugt ist ein Nachweisverfahren, das mit einem Diazoniumsalz durchgeführt wird, das sich von 1-Aminoanthrachinon oder von einem Anilin der Formel III ableitet, worin X¹ Wasserstoff, Chlor oder Nitro, X² Chlor oder Nitro und X³ Wasserstoff bedeuten. Besonders hervorzuheben ist ein Nachweisverfahren, das mit einem Diazoniumsalz durchgeführt wird, das sich von 1-Aminoanthrachinon ableitet.

Zur Nachweisreaktion verwendet man in der Regel eine wäßrige Lösung eines Diazoniumsalzes, die 0,1 bis 2 Gew.-%, bezogen auf das Gewicht der Lösung, an Diazoniumsalz aufweist. Je Gewichtsteil markiertes Mineralöl werden dabei im allgemeinen 0,001 bis 0,1 Gew.-Teile Diazoniumsalzlösung angewandt.

Wie oben bereits ausgeführt, ist es auch möglich, das Anilin der Formel I mittels eines wäßrigen Mediums, insbesondere mittels wäßriger Säure, die gegebenenfalls noch ein wassermischbares organisches Lösungsmittel enthält, aus dem Mineralöl zu extrahieren und mit einem obengenannten Diazoniumsalz, das sich in festem Aggregatzustand auf einem Träger befindet und im wäßrigen Extrakt gelöst wird, unter Bildung eines Azofarbstoffs zu kuppeln. Das wassermischbare organische Lösungsmittel kann für den erleichterten Übergang des Anilins I in die wäßrige Phase von Vorteil sein. Bei diesem Nachweis ist besonders auf die Einhaltung eines optimalen pH-Werts zu achten. Dies geschieht zweckmäßig mittels einer Puffersubstanz.

Besonders geeignet als Träger ist ein Papierstreifen, z.B. aus Filterpapier. Er kann mit einer Lösung eines der obengenannten Diazoniumsalze imprägniert und getrocknet werden. (Durch Lagerung der imprägnierten Papierstreifen im Trockenen und im Dunkeln kann dabei die Zersetzung des Diazoniumsalzes verhindert werden).

Wenn man einen solchen imprägnierten Papierstreifen in den wäßrigen Extrakt eintaucht, kommt es an seiner Oberfläche durch die Bildung eines Azofarbstoffs zu einer Farbreaktion. Auf diesem Weg können die Aniline der Formel I auf äußerst einfache Weise nachgewiesen werden.

Eine besonders vorteilhafte Methode besteht darin, zum wäßrigen Extrakt einige Schnipsel des imprägnierten Papierstreifens zu geben und gegebenenfalls kurzzeitig zu erwärmen.

Die Aniline der Formel I können in den Mineralölen natürlich auch noch mittels an sich bekannten physikalischen Analysemethoden nachgewiesen werden, beispielsweise mittels Gaschromatographie, Hochdruckflüssigkeitschromatographie, Dünnschichtchromatographie oder Säulenchromatographie.

Bei den erfindungsgemäß als Markierungsmittel für Mineralöle zur Anwendung gelangenden Aniline der Formel I handelt es sich um übliche an sich bekannte Produkte aus der Farbstoffherstellung. Sie sind leicht zugänglich.

Sie können noch in sehr kleinen Mengen im Mineralöl nachgewiesen werden und ergeben bei ihrem Nachweis eine starke Farbreaktion.

Die folgenden Beispiele sollen die Erfindung näher erläutern.

### Beispiel 1

### a) Herstellung der Reagenzlösungen A, B und C

Reagenzlösung A
   Der feuchte Preßkuchen des schwefelsauren Diazoniumsalzes von 1-Aminoanthrachinon (Feststoffgehalt: 73 Gew.-%) wurde 2,5 gew.-%ig in Wasser gelöst. Der pH-Wert der Lösung betrug ca. 1,2.
Reagenzlösung B
   Durch wäßrige Diazotierung von 2-(3-Phenyl-1,2,4-oxdiazol-5-yl)anilin mit Natriumnitrit erhielt man eine 0,063 molare Diazoniumchloridlösung. Der pH-Wert der Lösung war ca. 0,1.
Reagenzlösung C
   Durch wäßrige Diazotierung von 2-Chlor-4-nitroanilin mit Natriumnitrit erhielt man eine 0,208 molare Diazoniumchloridlösung. Der pH-Wert der Lösung war 0.

### b) Allgemeine Nachweisreaktion in saurer Lösung

Das jeweilige Anilin wurde in einer Konzentration von 10 ppm in Dieselkraftstoff gelöst. 10 ml dieser Lösung wurden mit 0,1 ml der obenbeschriebenen Reagenzlösung A 1 min intensiv geschüttelt. Danach wird das Gemisch mit 3 ml 9 gew.-%iger Salzsäure nochmal 1 min geschüttelt. Nach dem Absetzen ist die untere wäßrige Phase gefärbt.

Zur photometrischen Messung wird die wäßrige Phase mit destilliertem Wasser auf 10 % der ursprünglichen vorhandenen Konzentration verdünnt.

### c) Allgemeine Nachweisreaktion in gepufferter wäßriger Lösung

Es werden 10 g des mit 10 ppm markierten Dieselkraftstoffs mit 1 g 9 gew.-%iger Salzsäure extrahiert, die wäßrige Phase mit Natronlauge neutralisiert und mit jeweils der vierfachen Menge einer der drei im folgenden genannten Pufferlösungen 1, 2 und 3 versetzt.

Pufferlösung 1: 5 gew.-%ige wäßrige Citronensäurelösung mit 10 Gew.-%iger Natronlauge auf einen pH-Wert von pH 3,3 eingestellt.

Pufferlösung 2: 5 gew.-%ige wäßrige Kaliumdihydrogenphosphatlösung mit einem pH-Wert von 4,4.

Pufferlösung 3: 5 gew.-%ige wäßrige Natriumacetat-trihydratlösung mit einem pH-Wert von 8,2.

Anschließend gibt man 0,1 g einer der drei Reagenzlösungen A, B oder C zu.

Die Kupplung läuft meist recht schnell ab, jedoch kann sie durch kurzzeitiges Erwärmen in den meisten Fällen weiter beschleunigt werden.

Die Ergebnisse der Untersuchungen sind in der folgenden Tabelle aufgeführt. Die Intensität der Färbung wird durch Noten (von 1 bis 5) beurteilt (1: keine Färbung, 5: sehr intensive Färbung). Außerdem wird angegeben, wenn die Farbreaktion sofort eintritt.

### d) Herstellung des Reagenzpapiers

5 g des unter "Reagenzlösung A" genannten Preßkuchens des Diazoniumsalzes von 1-Aminoanthrachinon wurden in 95 ml destilliertem Wasser im Ultraschallbad gelöst und die Lösung über ein Faltenfilter abfiltriert. In diese Lösung wurden Streifen von Filterpapier getaucht, die überschüssige Lösung entfernt und die nassen Streifen im Dunkeln zum Trocknen aufbewahrt. Bei Lagerung dieser Papiere im Dunkeln trat auch nach mehreren Wochen keine Verringerung der Reaktivität auf, allerdings verfärbte sich das Diazopapier bräunlich. Beim Lagern bei 50°C über 8 Tage war die Verfärbung des Papiers zwar erheblich, jedoch waren auch diese Papiere noch zum Farbtest geeignet.

### e) Nachweis mittels Reagenzpapier

10 ml Heizöl, das mit 10 ppm N,N-[Bis(2-hydroxyethyl)]-3-methylanilin (siehe Beispiel 3) markiert war, wurden mit 1 g 4 gew.-%iger Salzsäure im Reagenzglas versetzt und von Hand 1 min kräftig geschüttelt. Nach erfolgter Phasentrennung wurde die untere wäßrige Phase für die nachfolgenden Detektionsversuche verwendet.

1 g dieser wäßrigen Phase wurde mit 0,55 ml 33 gew.-%iger wäßriger Natriumacetat-trihydrat-Lösung versetzt. Der pH-Wert dieser Lösung lag bei 4,5. Ein Tropfen dieser Lösung wurde auf das "Diazopapier" gegeben, das sofort eine rote Färbung zeigte.

Anstelle des Auftropfens auf das "Diazopapier" kann auch ein Streifen dieses Papiers in die Lösung gestellt werden. Dabei läuft die verdünnte aromatische Aminlösung auf dem Diazopapier und entwickelt den Farbstoff nur am Start, da dieser dann nicht mehr läuft. Durch weiteres Nachliefern von Anilin wird dieser Fleck immer stärker und so kann das Anilin auch noch in verdünnten Lösungen einwandfrei nachgewiesen werden. Dieser Aufkonzentrationseffekt ist besonders dann vorteilhaft, wenn bei einem "Straßentest" nur Spuren des Anilins nachgewiesen werden sollen.

Alternativ können auch Schnipsel des Diazopapiers in die Lösung gegeben werden. Das Diazoniumsalz löst sich heraus und die Aminlösung verfärbt sich schnell. In manchen Fällen muß die Lösung erhitzt werden.

### f) Vergleich (Nullprobe)

Zum Vergleich wurden 10 ml des unmarkierten Dieselkraftstoffs mit 0,1 ml der Reagenzlösung A 1 min intensiv geschüttelt. Danach wurde mit 3 ml 9 gew.-%iger Salzsäure nochmals kräftig durchgeschüttelt. Nach dem Absetzen erschien die wäßrige Phase, je nach Dieselkraftstoffsorte, mehr oder weniger stark gelblich gefärbt. Für die photometrische Auswertung (siehe b)) wurde die auf 10 % der ursprünglich vorhandenen Konzentration verdünnte wäßrige, fast farblose Lösung dieser Blindprobe in den Vergleichsstrahl gestellt.

Analog c) wurde die Nachweisreaktion auch in gepufferter wäßriger Lösung vorgenommen.

### Beispiel 2

a) Das Diazoniumhydrogensulfat von 1-Aminoanthrachinon wurde mit Titandioxid im Gewichtsverhältnis 1 : 1 gemischt und in einer Reibschale verrieben.
   Das so gebildete Pulver wurde dann mit soviel Wasser versetzt, daß eine 0,5 gew.-%ige Lösung des Diazoniumsalzes entstand. Nach Filtration wurde die Lösung in einem vor Licht geschützten Gefäß aufbewahrt.
b) 10 ml Dieselkraftstoff, der mit 10. ppm N-Ethyl-N-(2-hydroxyethyl)-3-methylanilin markiert war, wurde mit 1 Tropfen (0,05 ml) der unter a) beschriebenen Lösung, die mit deionisiertem Wasser nochmals auf die Hälfte der Konzentration (1 : 1) verdünnt war, versetzt. Das Gemisch wurde dann 1 min geschüttelt. Es bildeten sich zwei Phasen, wobei die untere (wäßrige) Phase deutlich blaustichig rot gefärbt war.

Bei einer Blindprobe mit unmarkiertem Dieselkraftstoff färbte sich die wäßrige Phase fast überhaupt nicht.

Auf diese Weise ist das Markierungsmittel auch noch in einer Konzentration von 1 ppm im Dieselkraftstoff nachweisbar.

Die hier beschriebene Nachweisreaktion ist bei allen Anilinen der Formel I anwendbar. Es ist außerdem auch möglich, auf diesem Weg die Aniline der Formel I quantitativ nachzuweisen (z.B. mittels HPLC oder photometrischer Bestimmung).

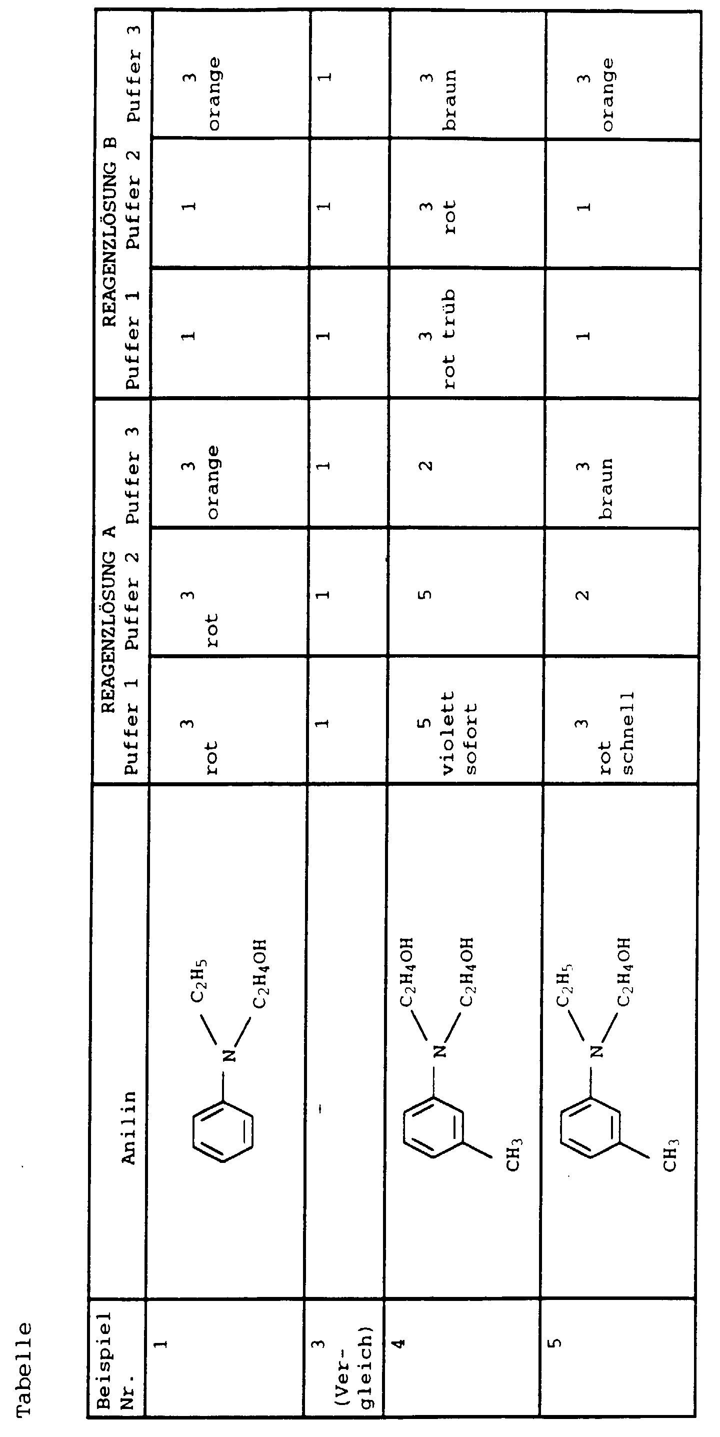

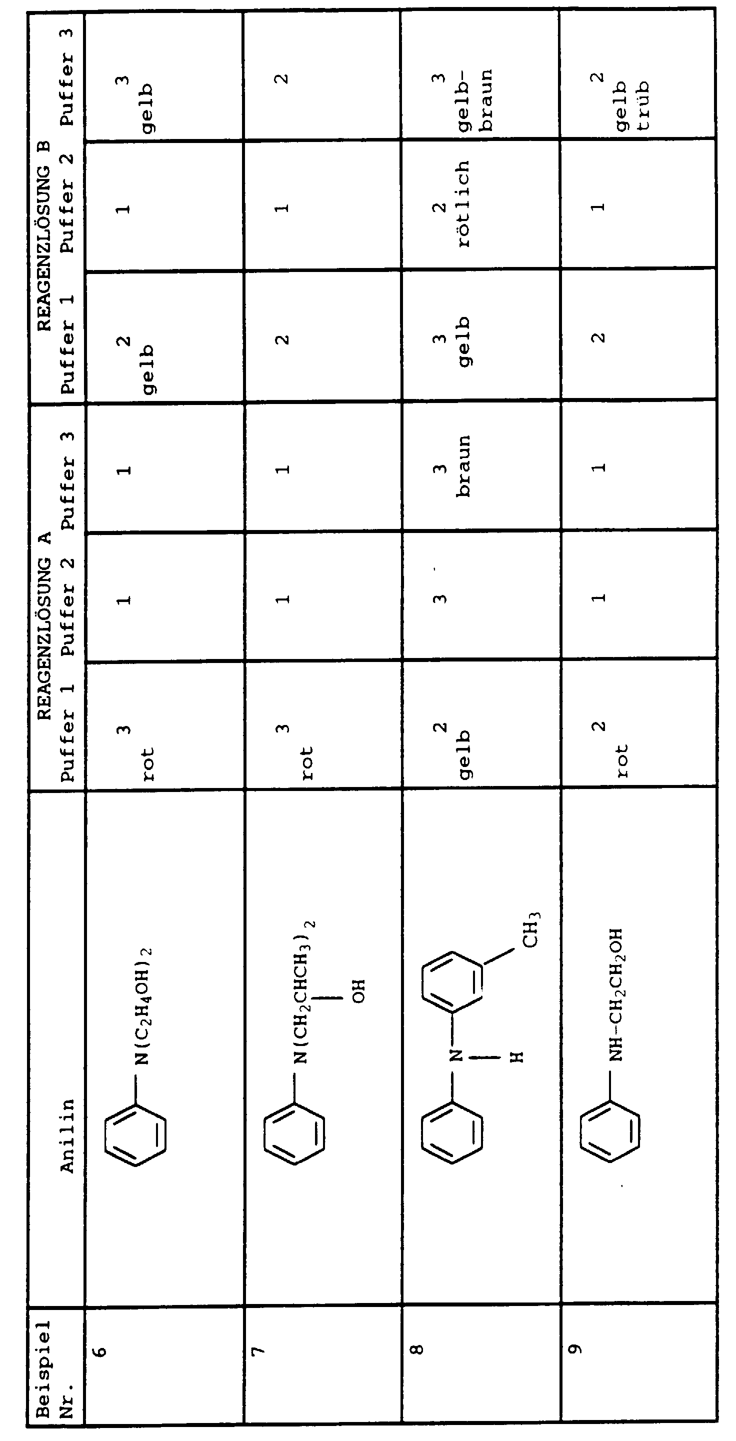

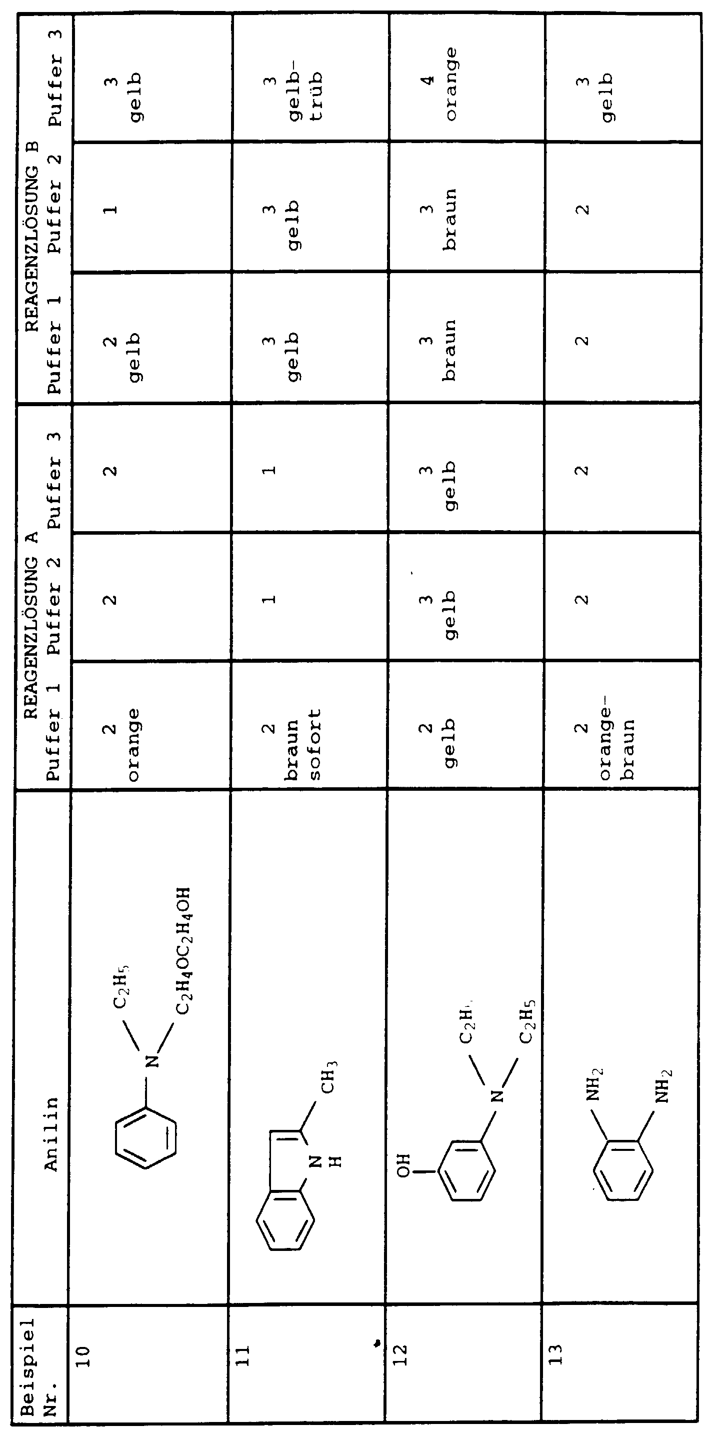

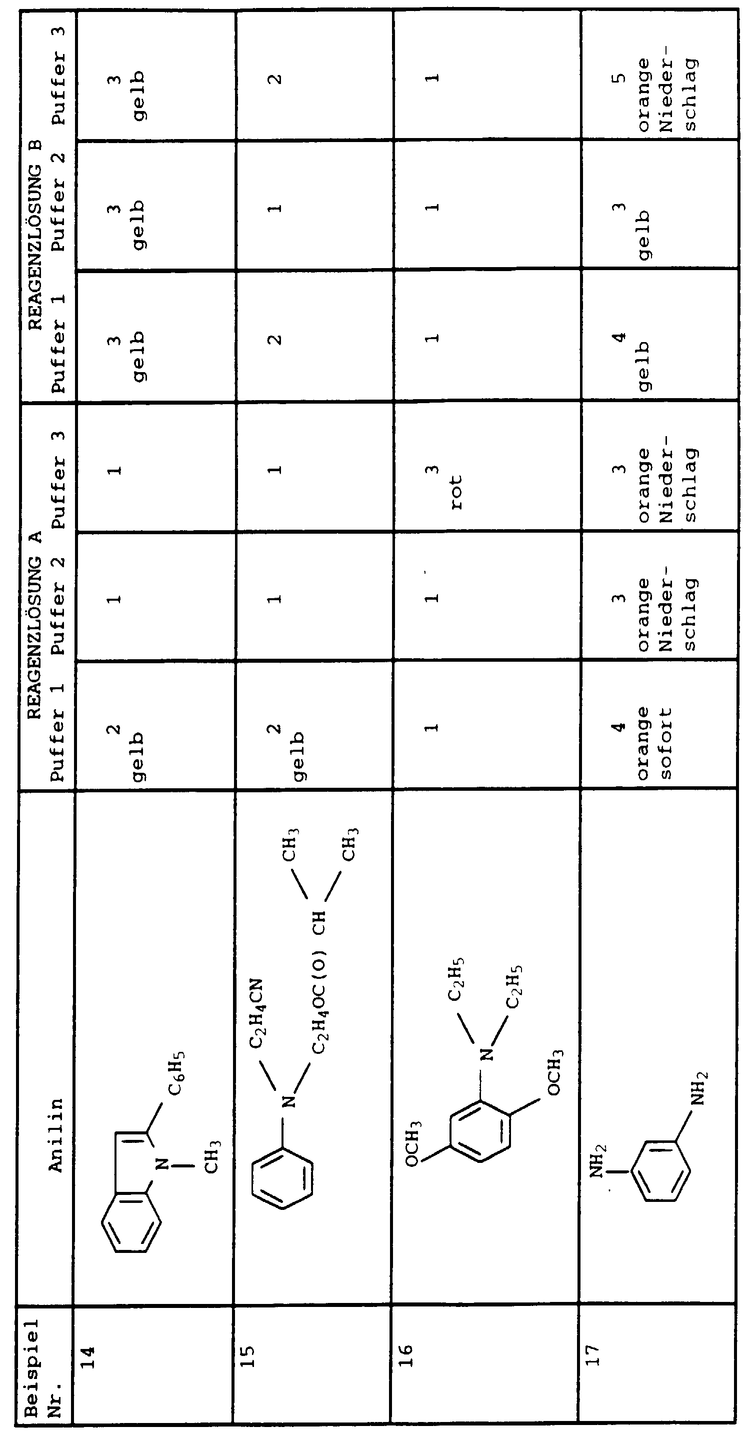

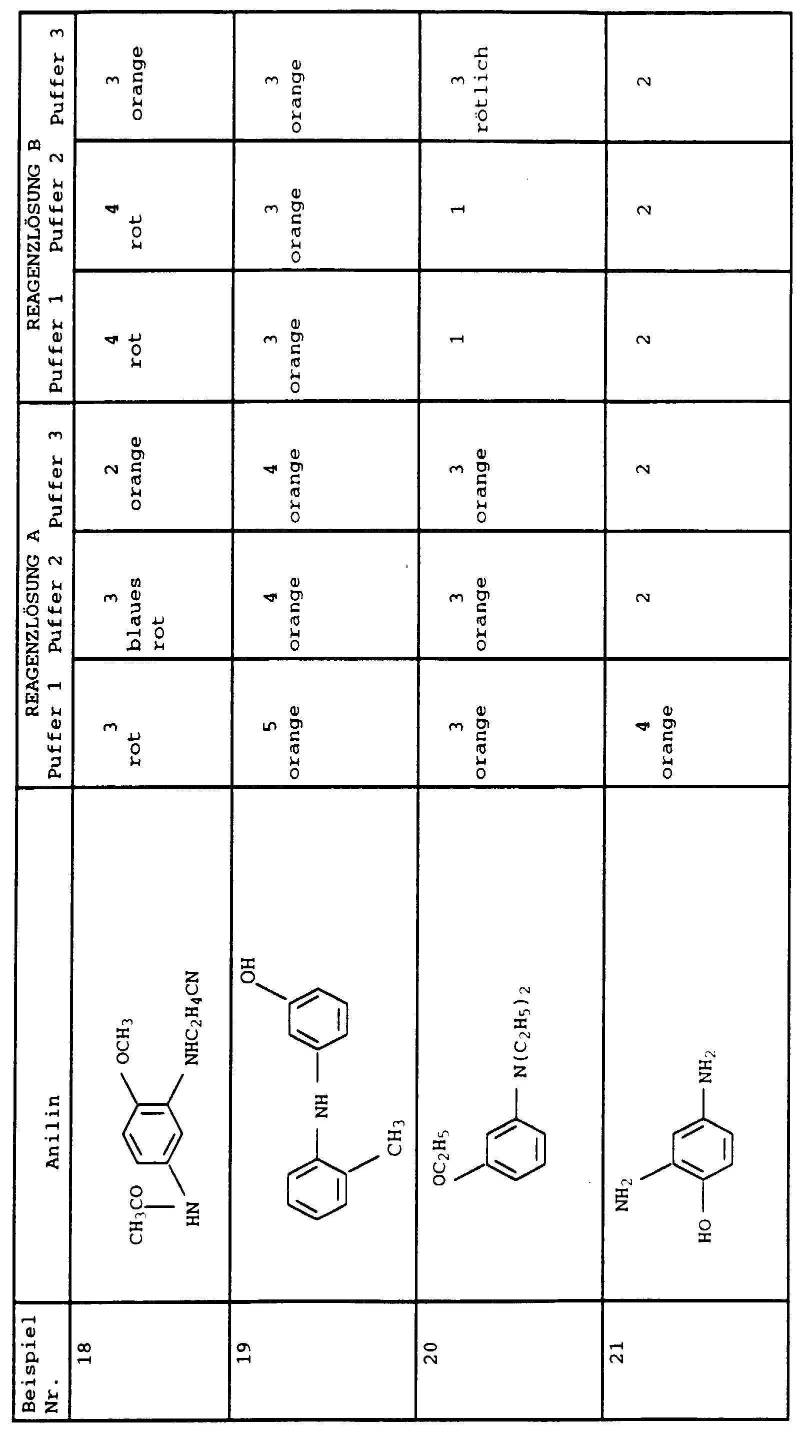

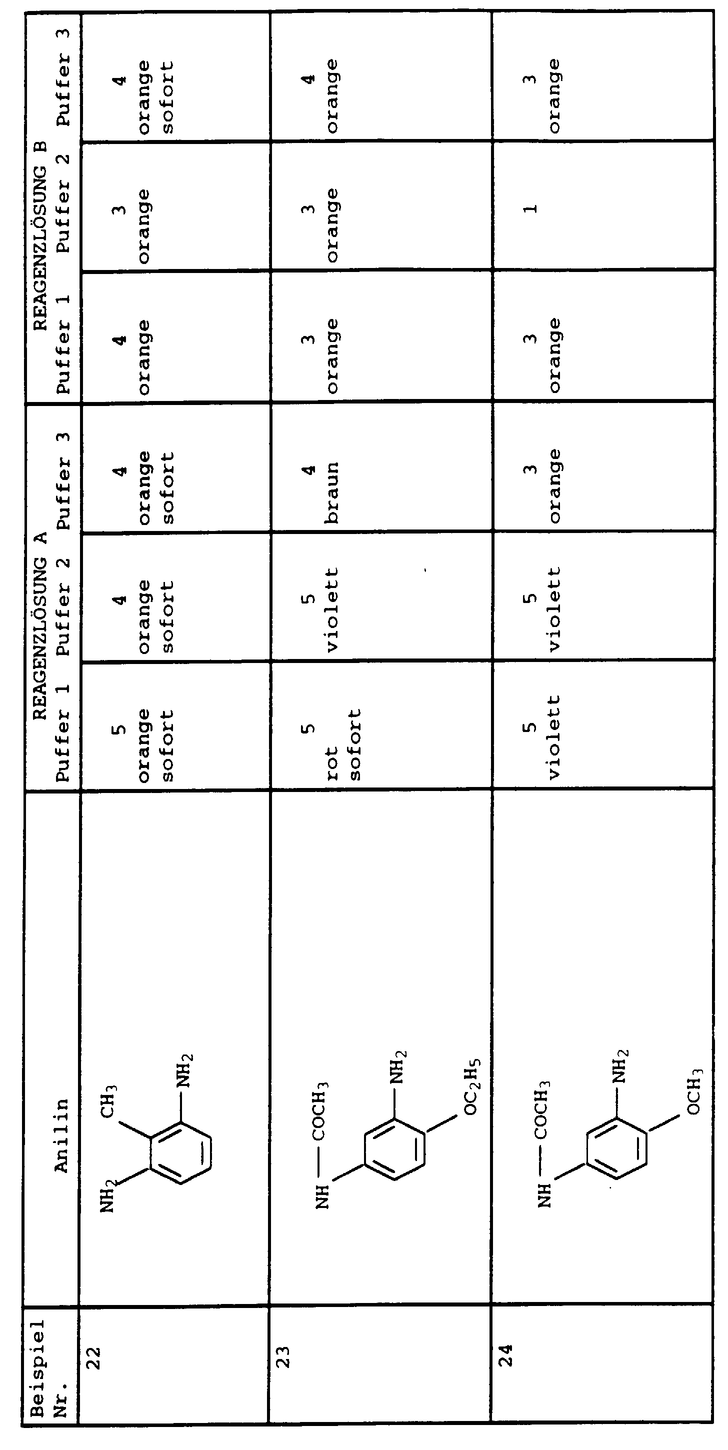

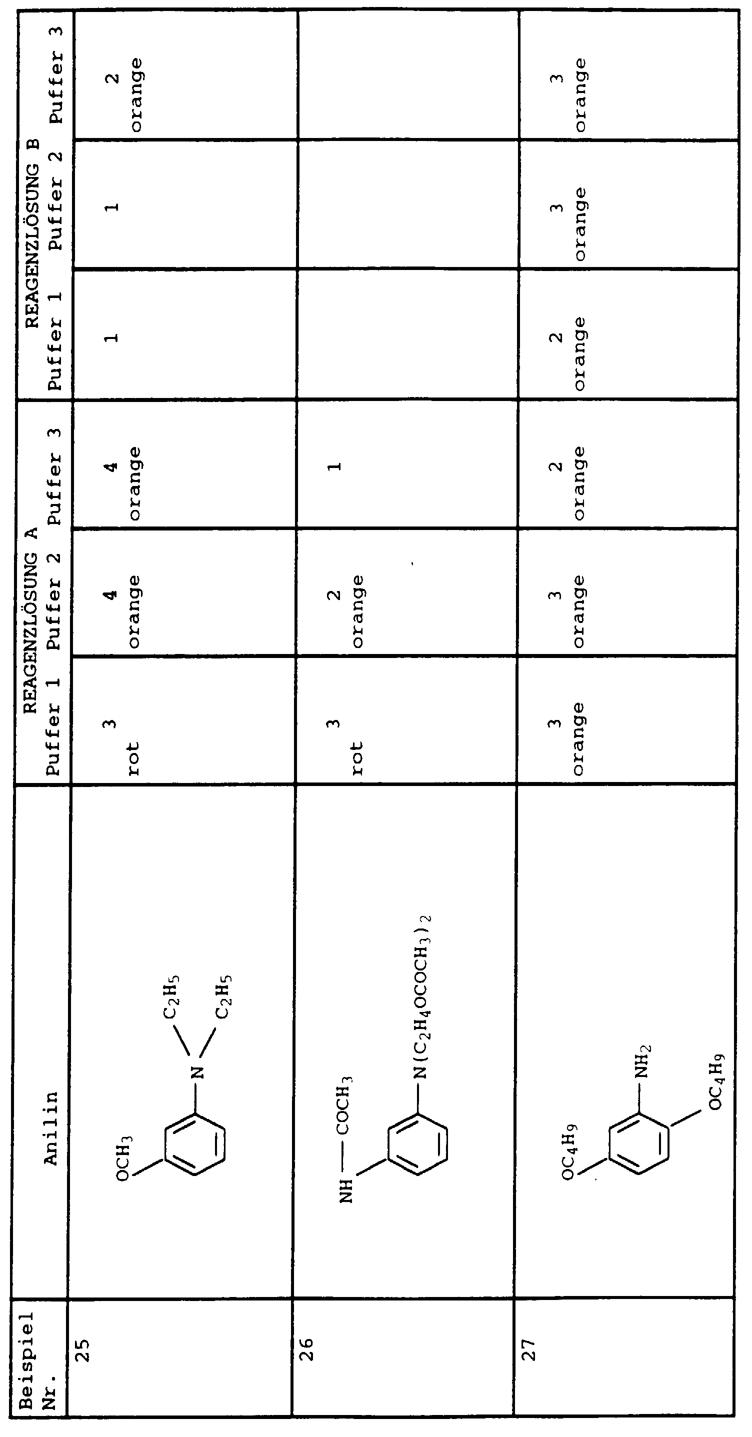

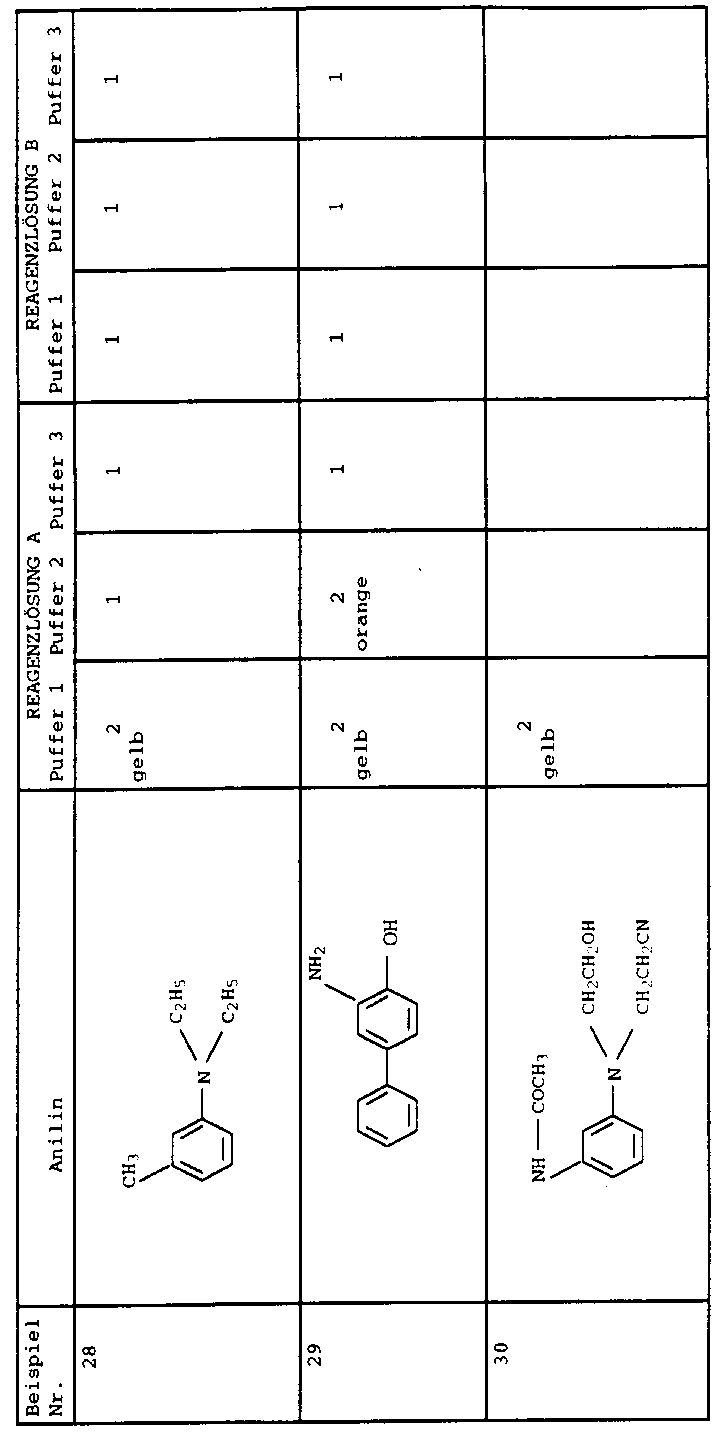

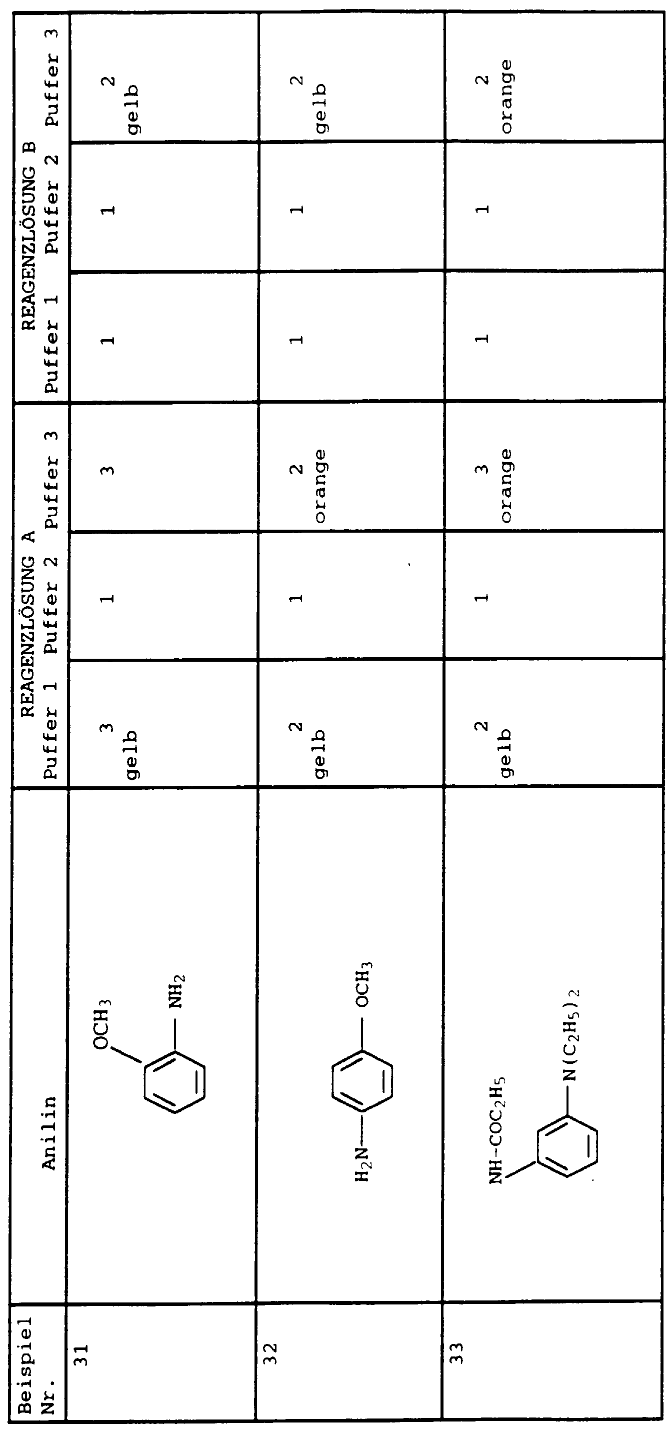

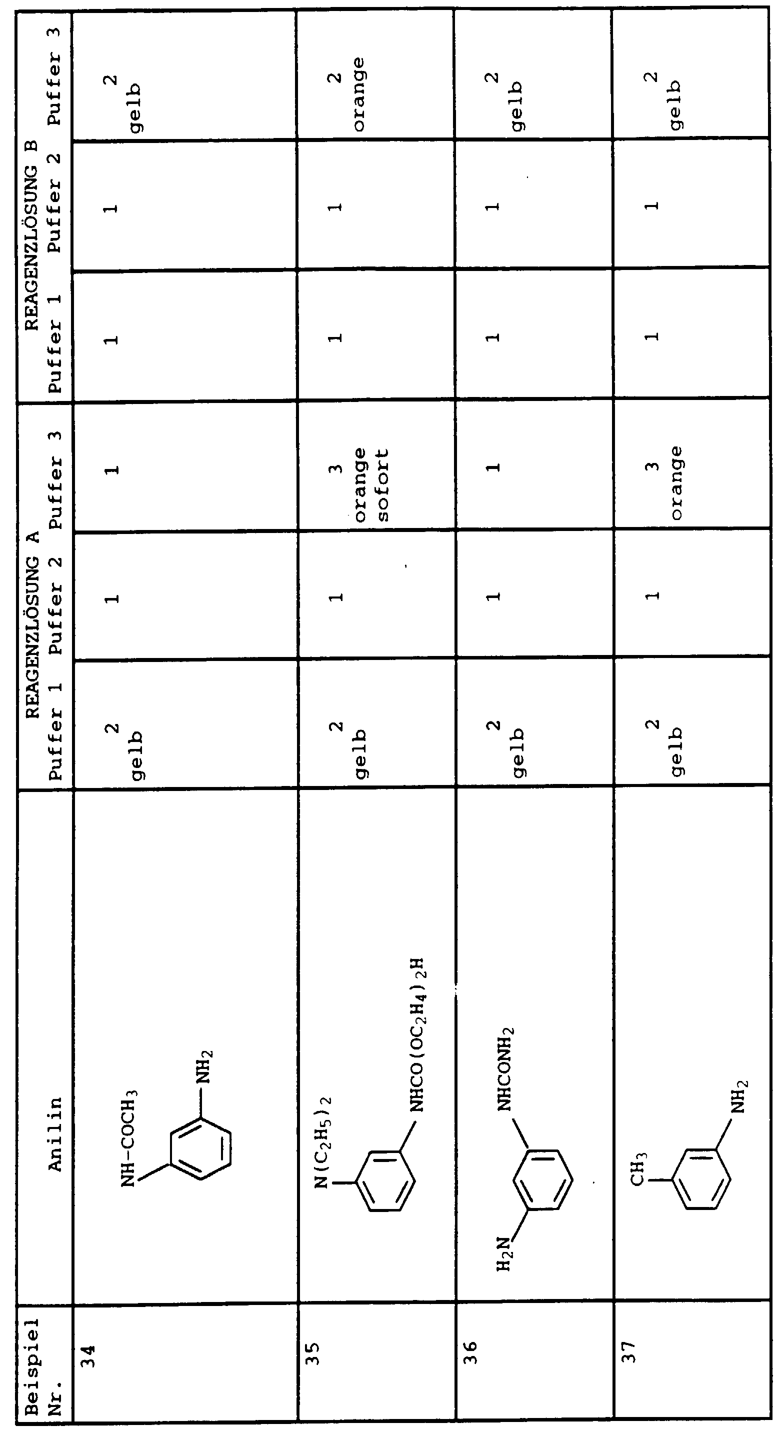

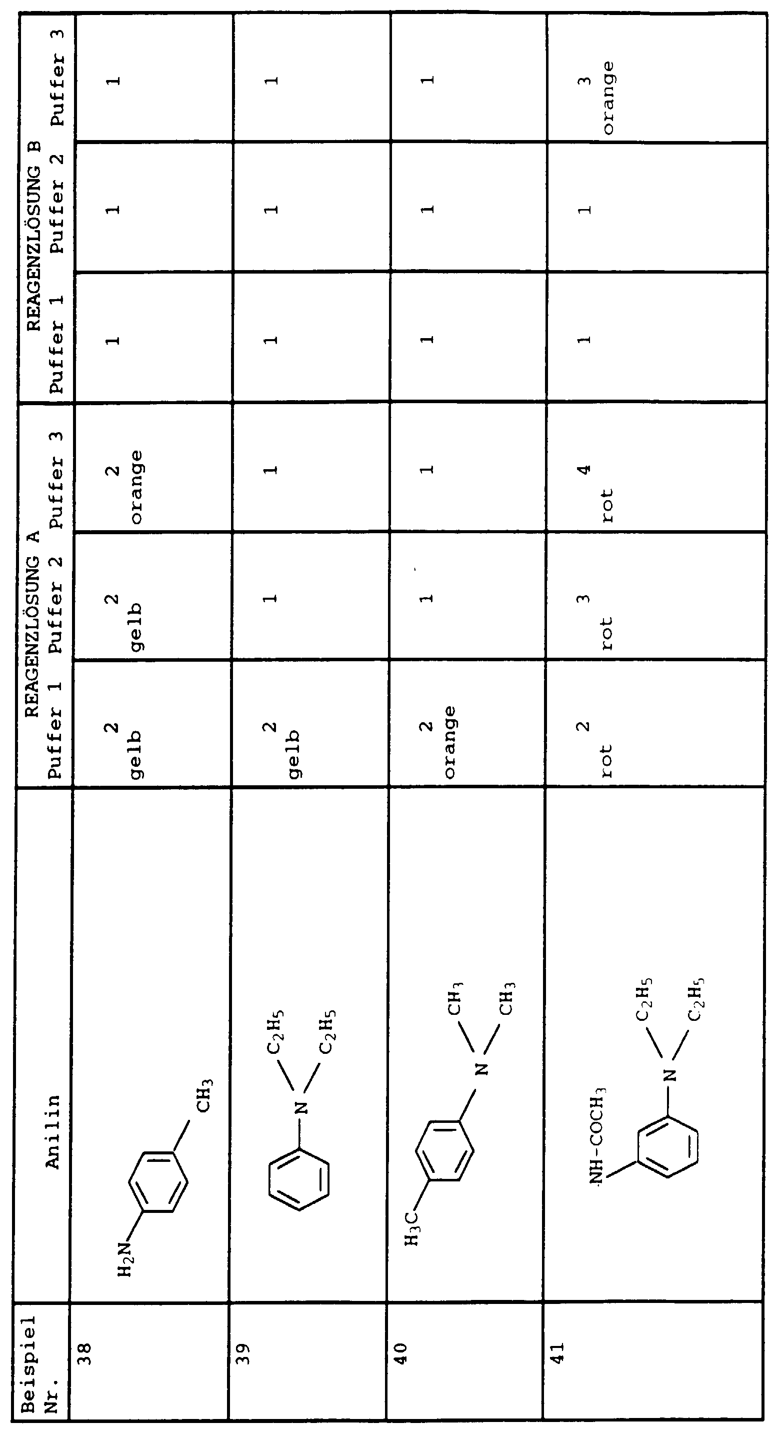

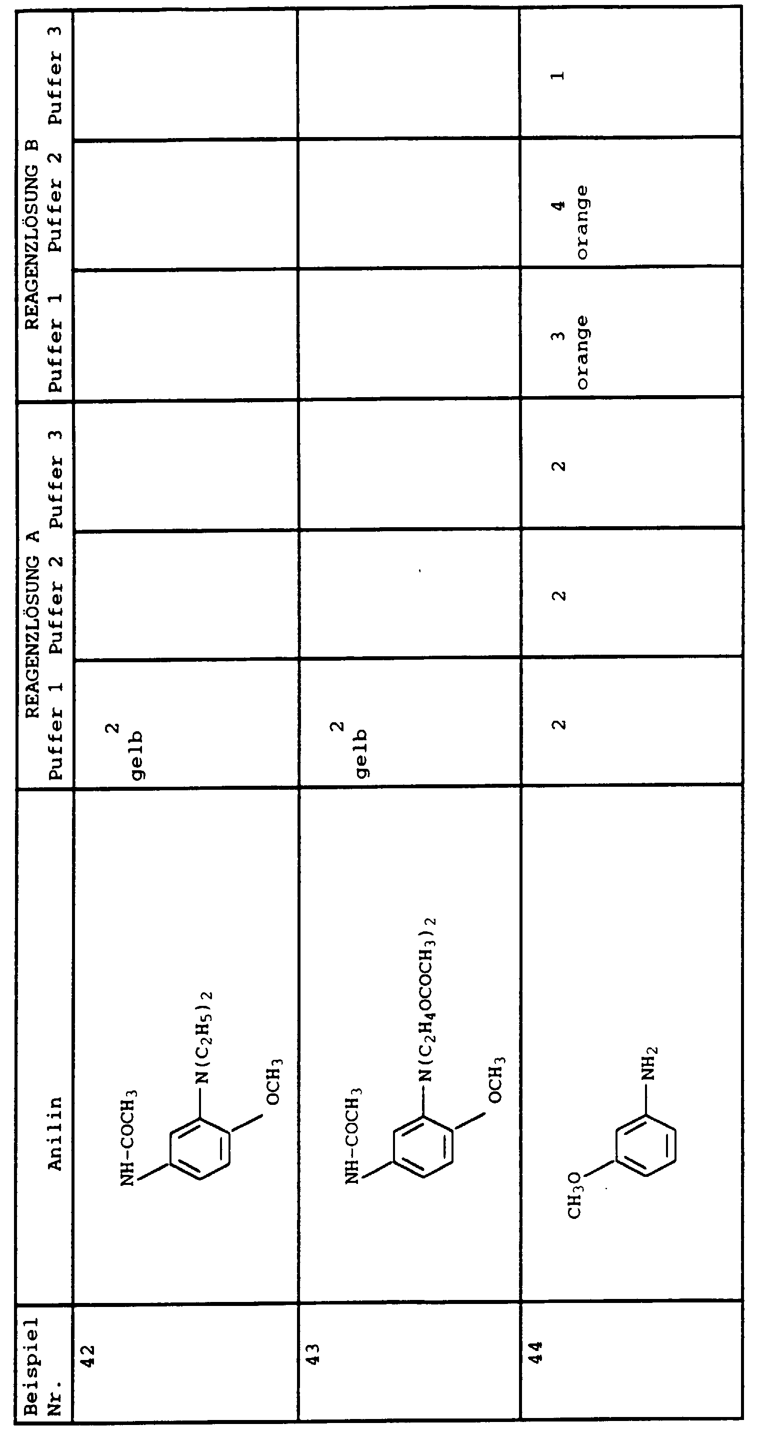

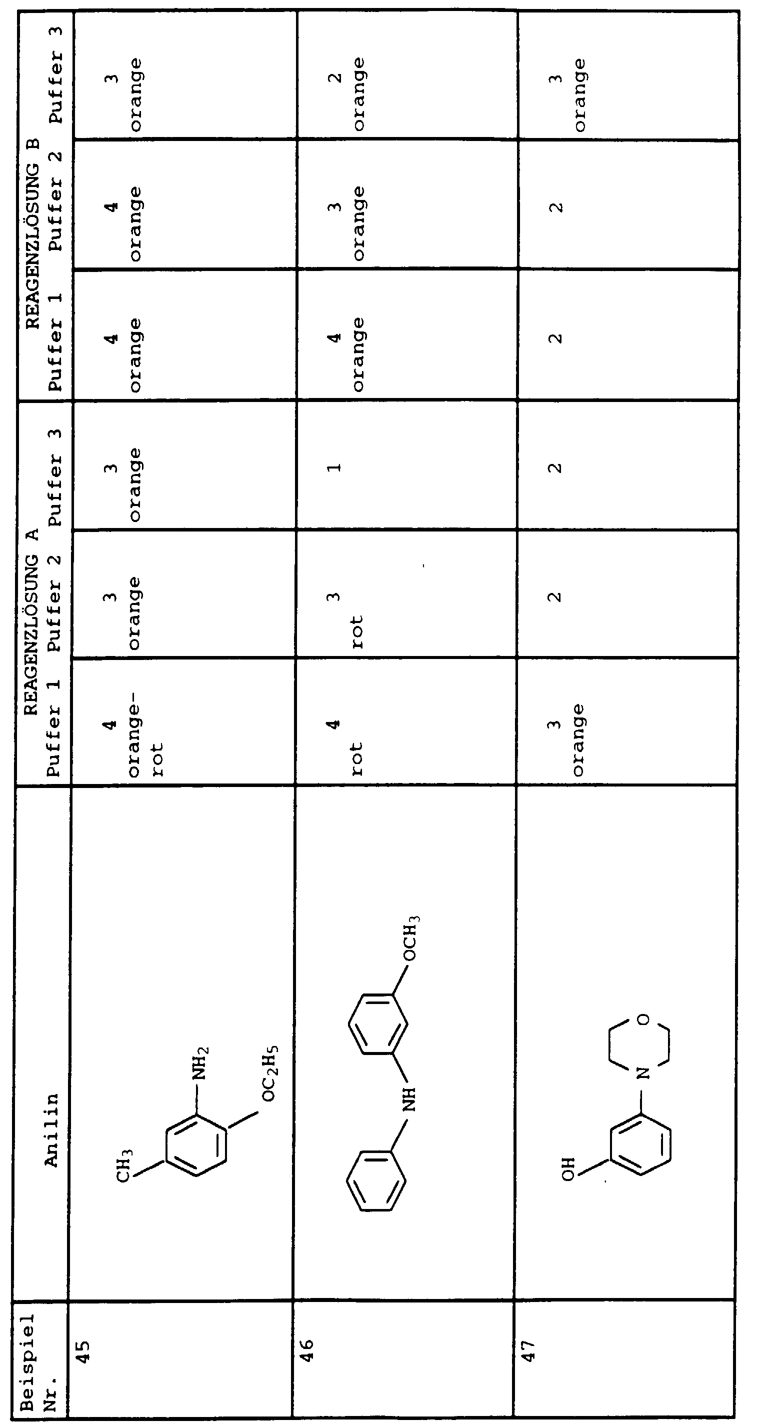

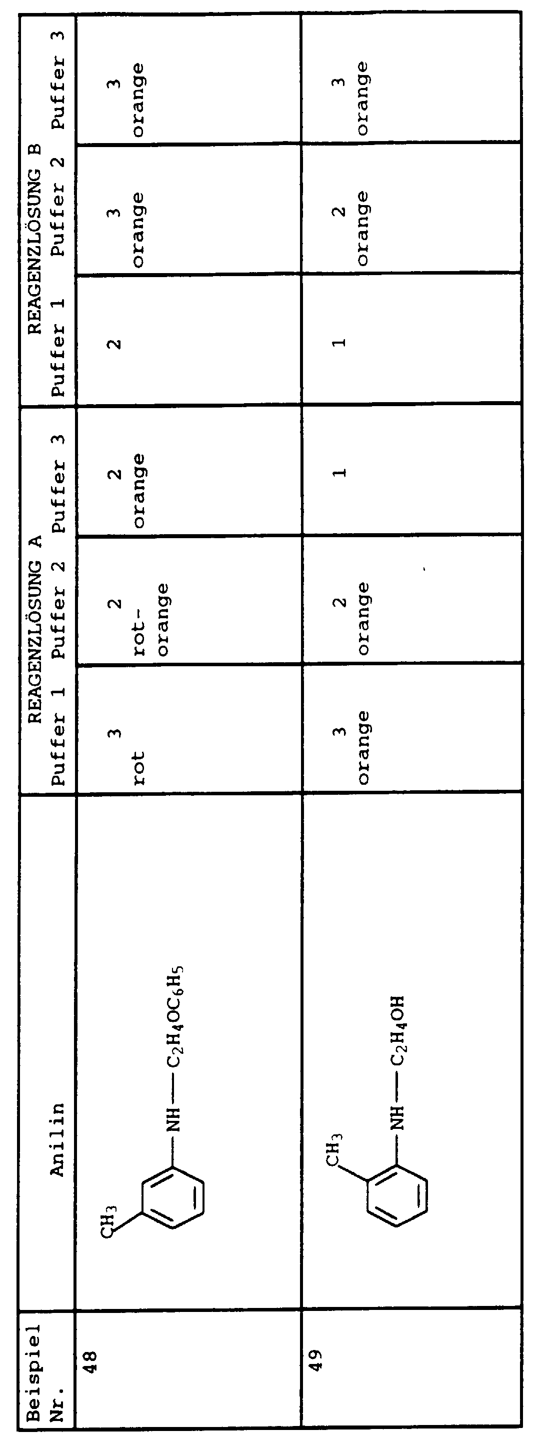

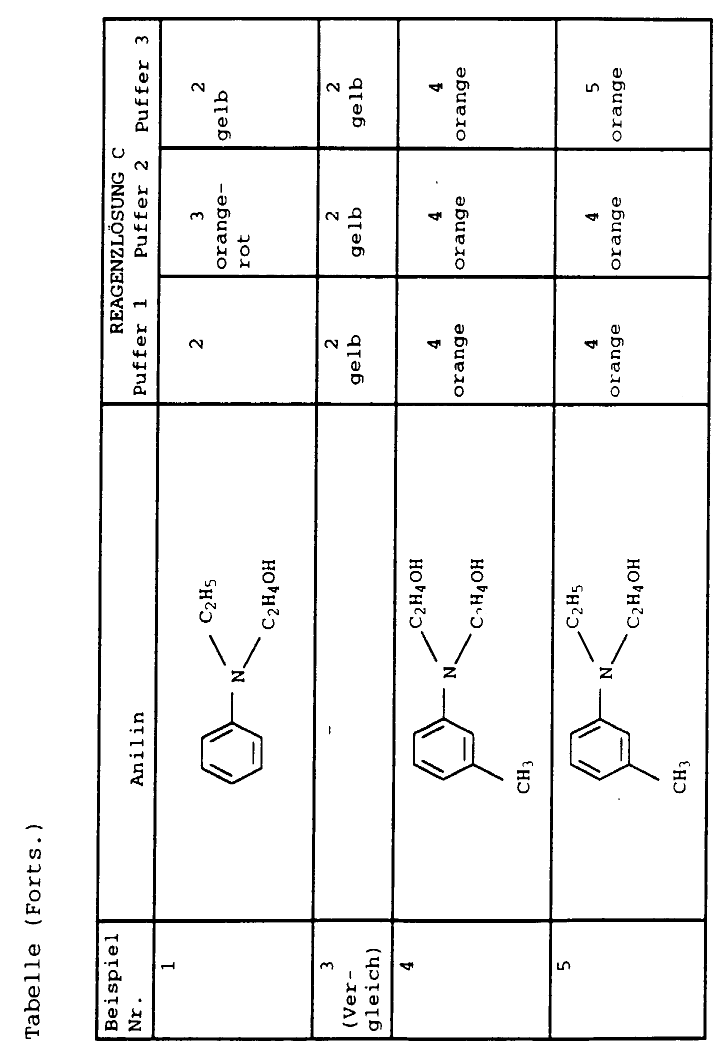

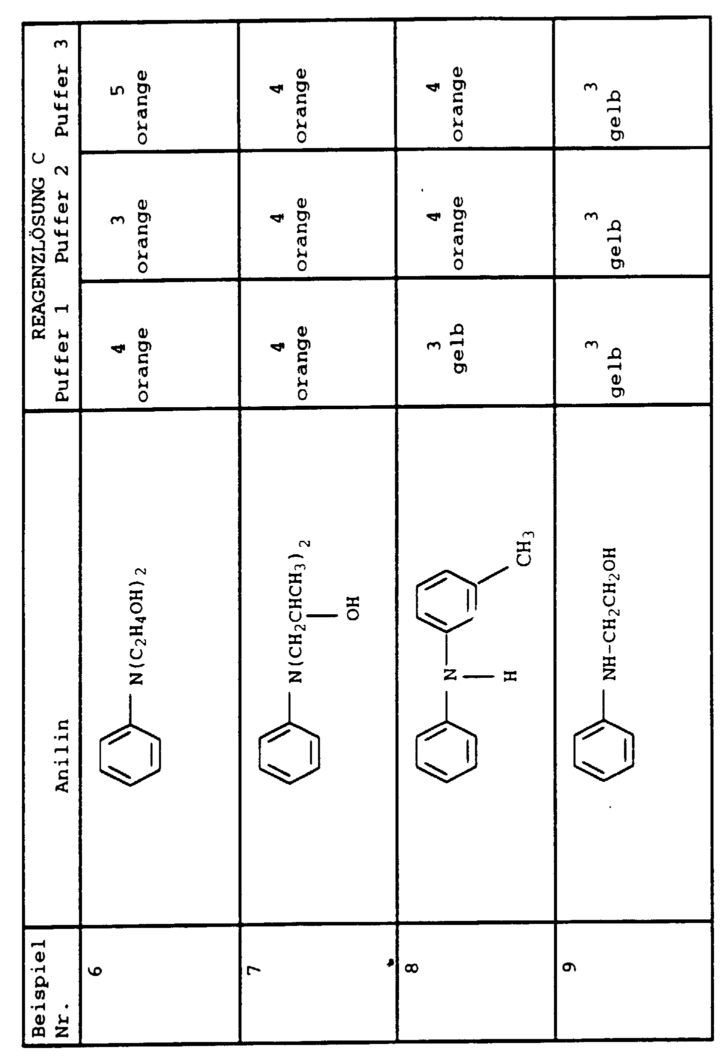

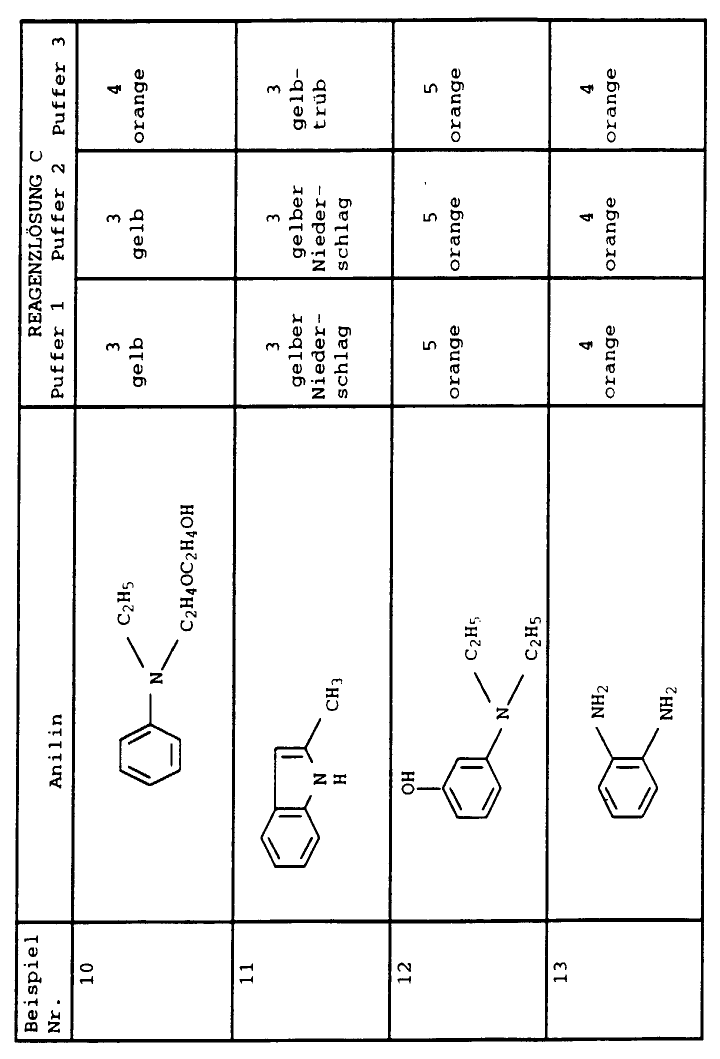

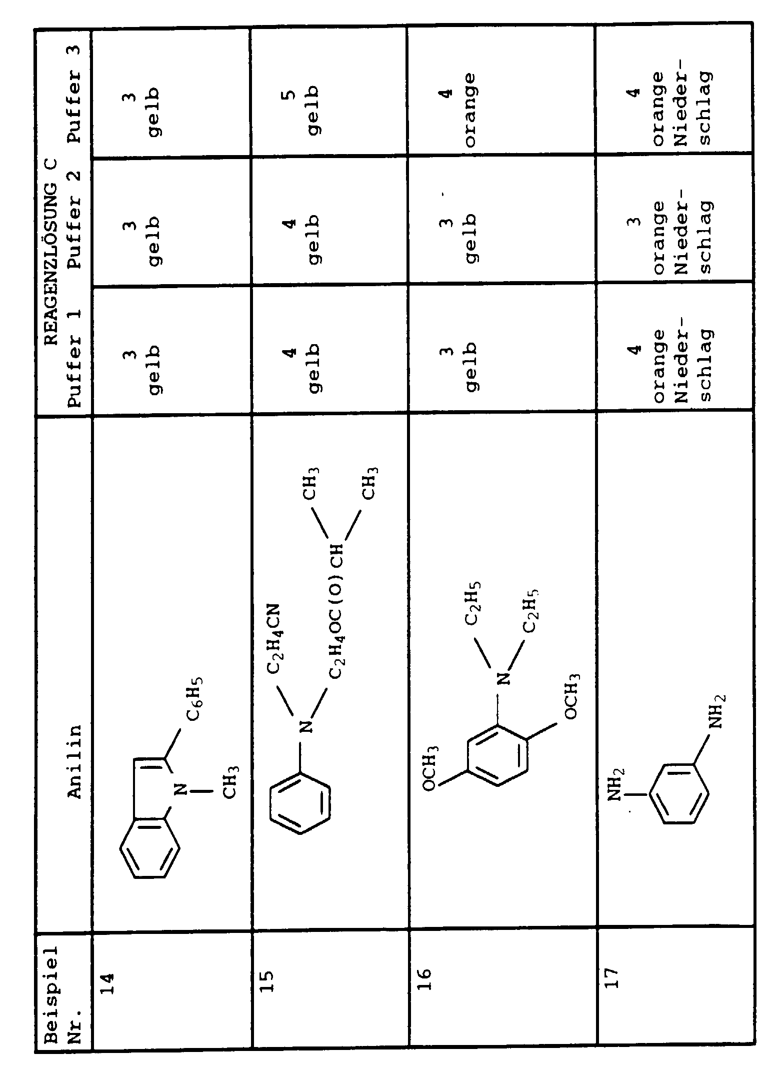

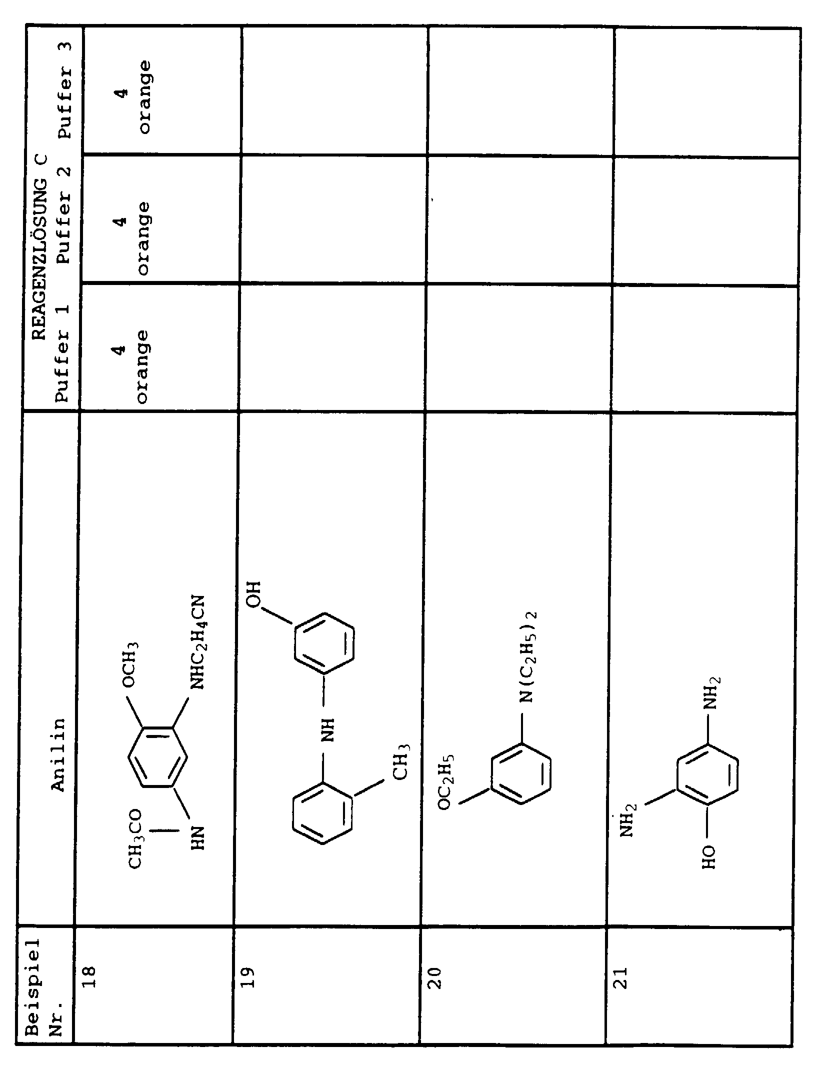

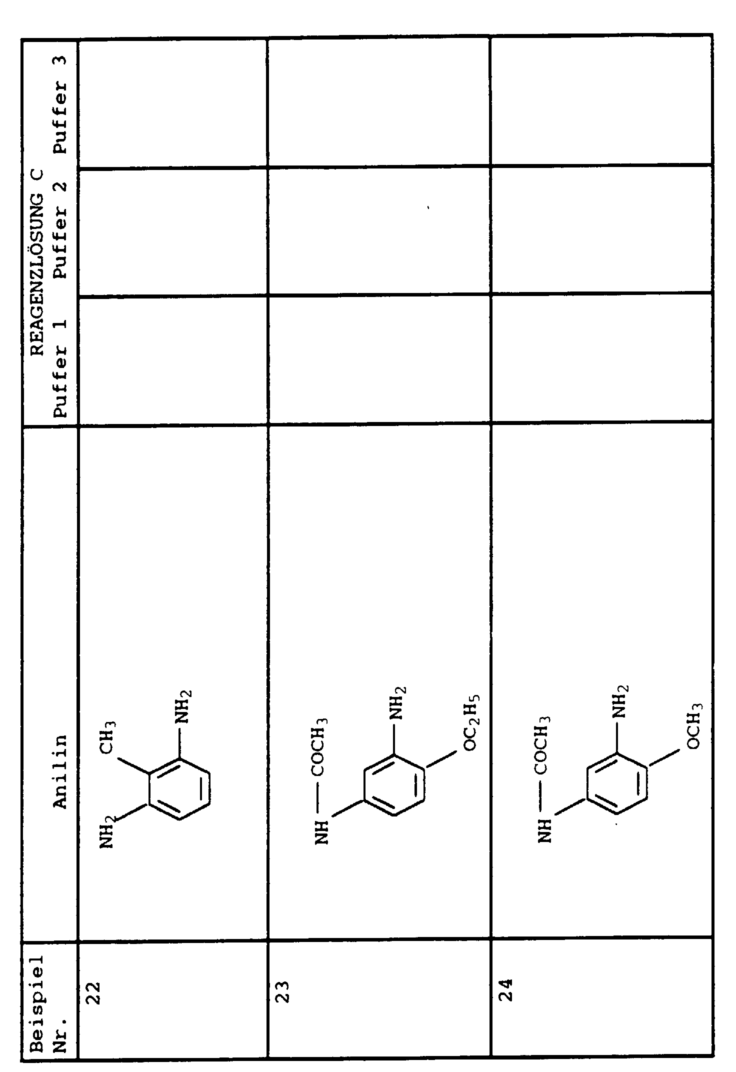

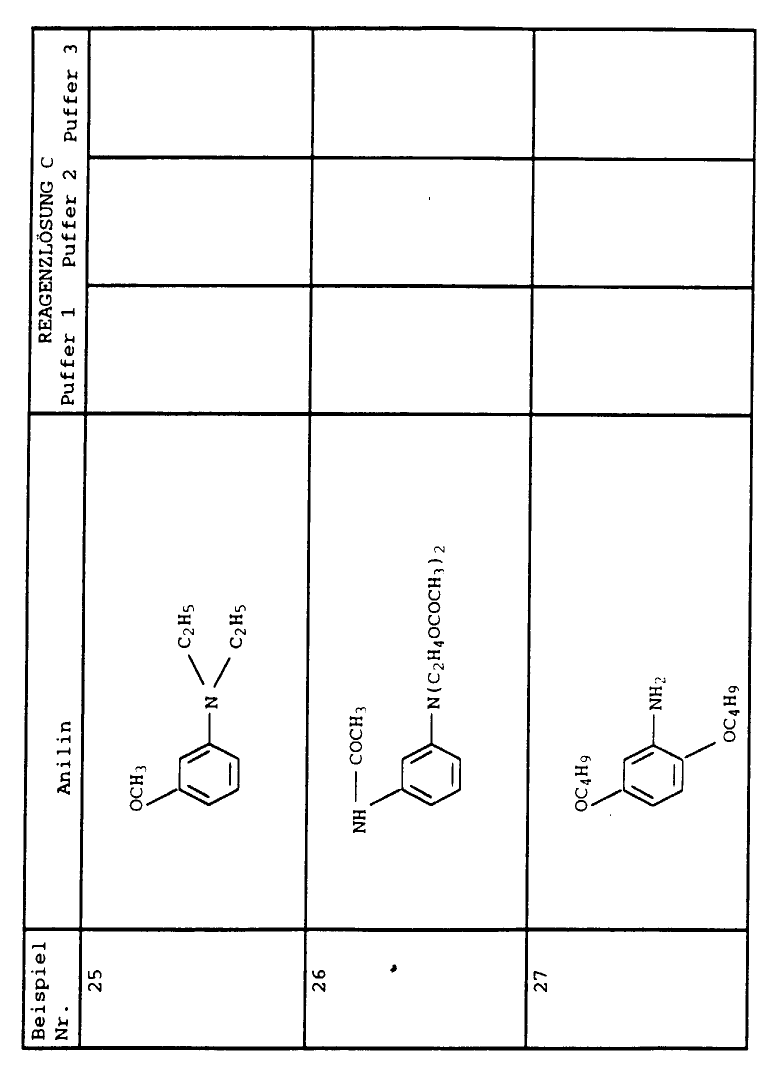

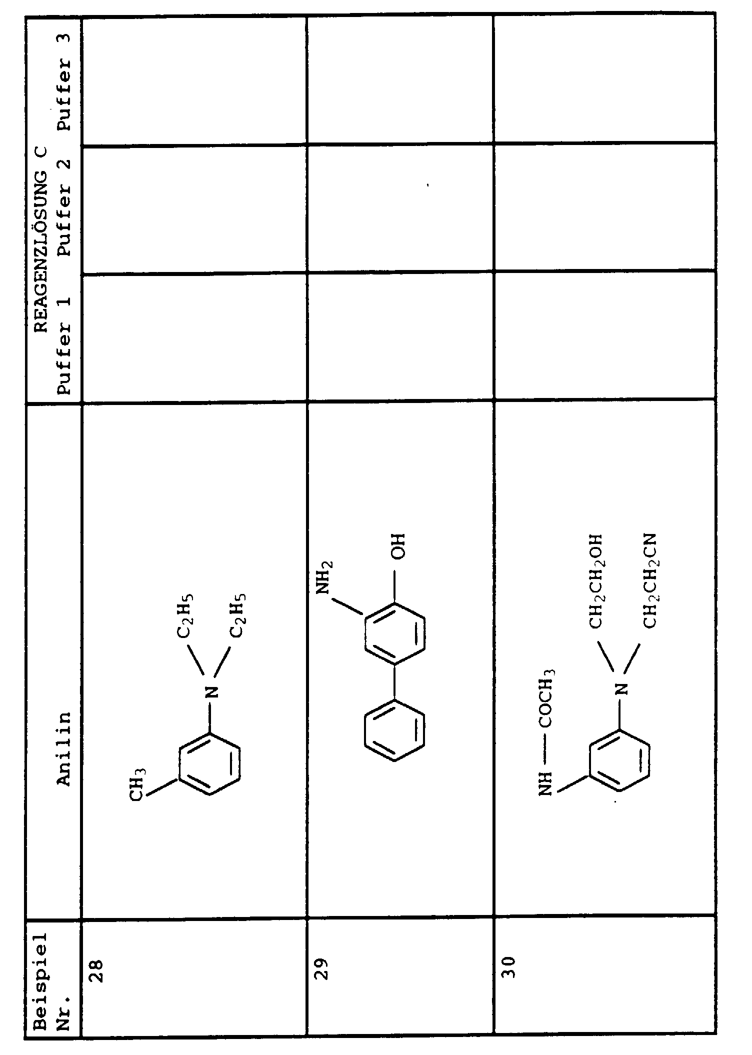

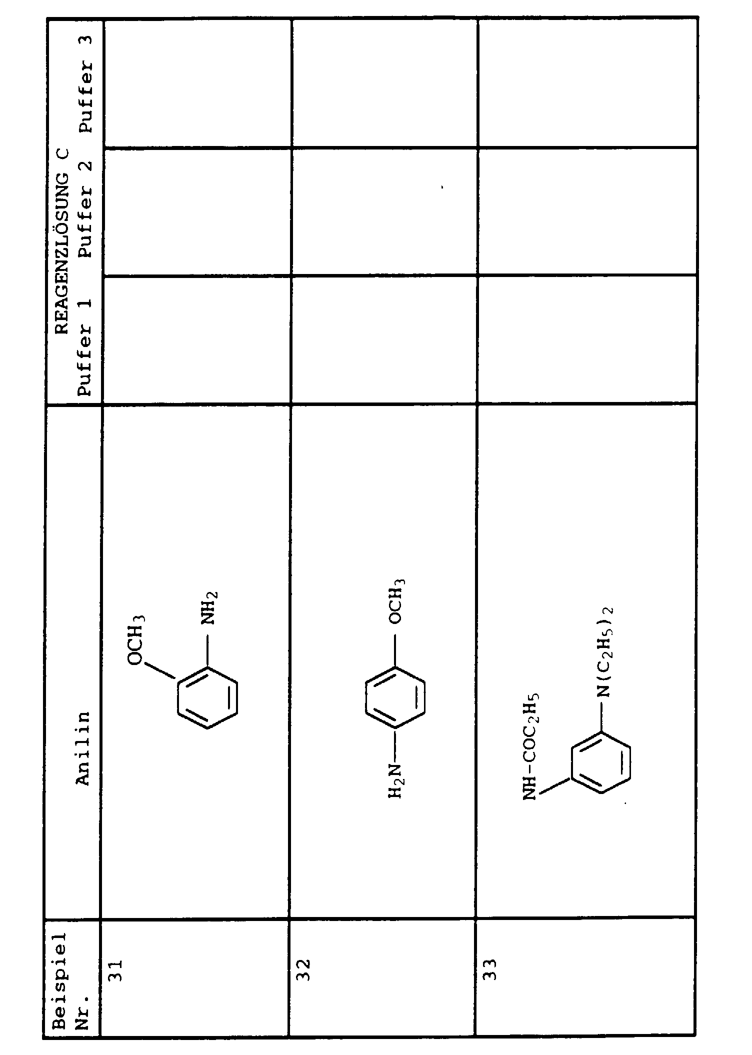

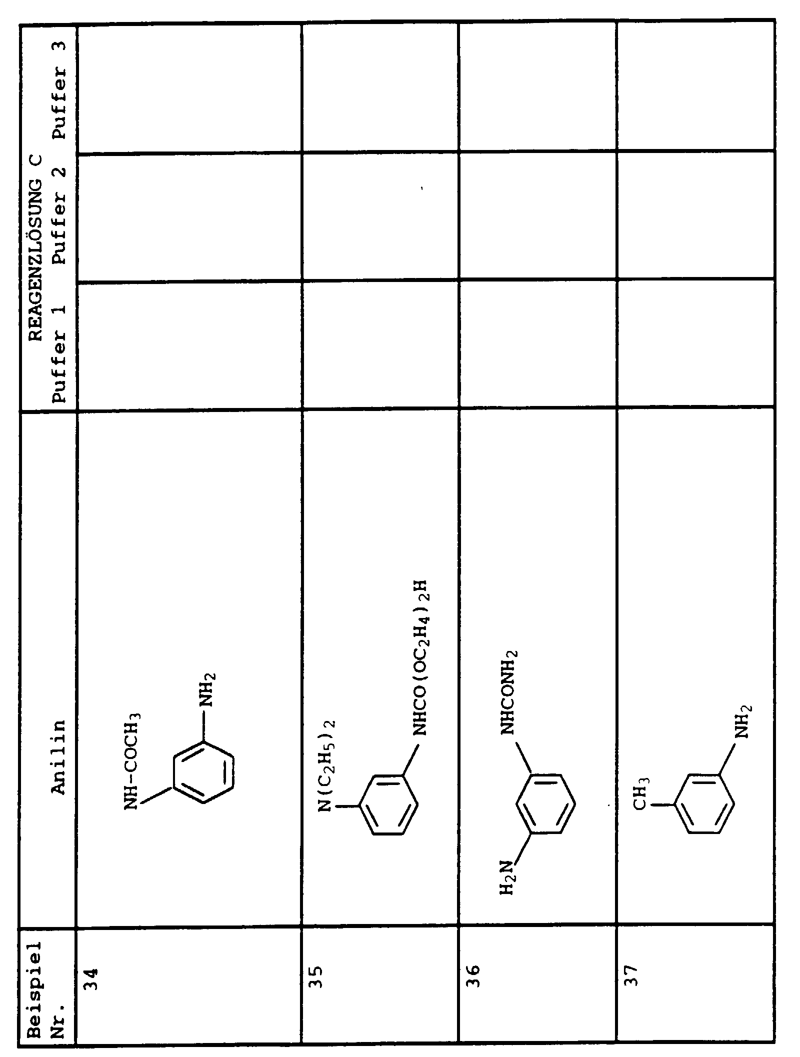

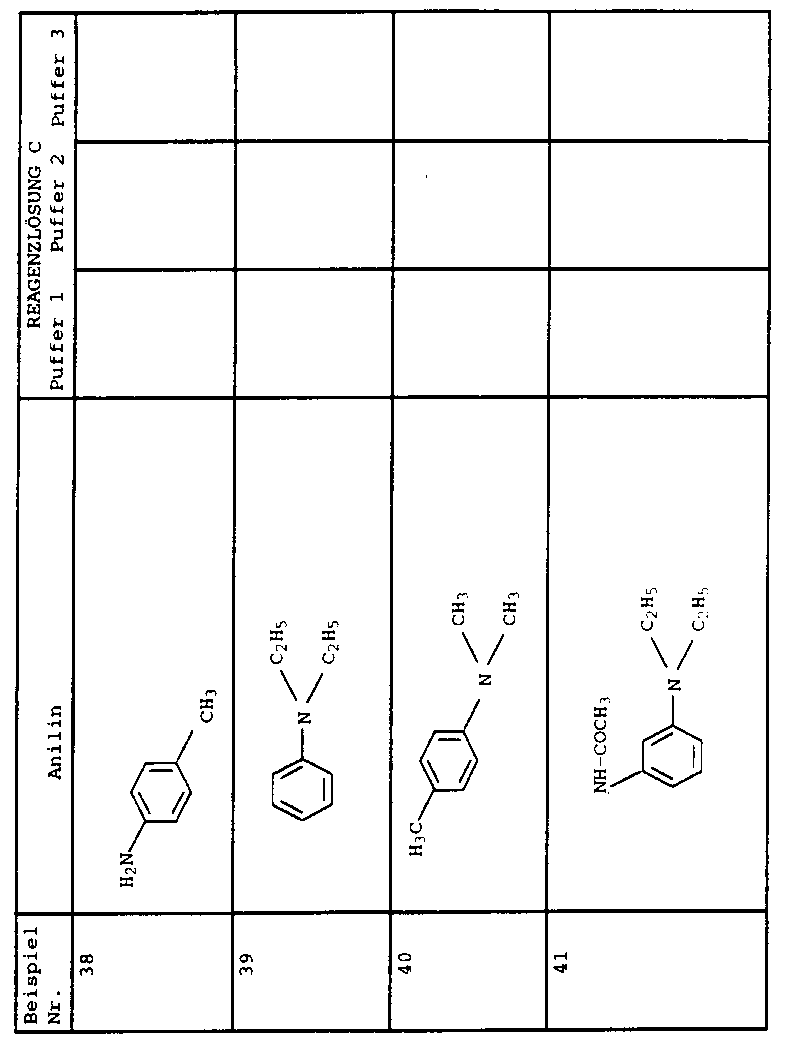

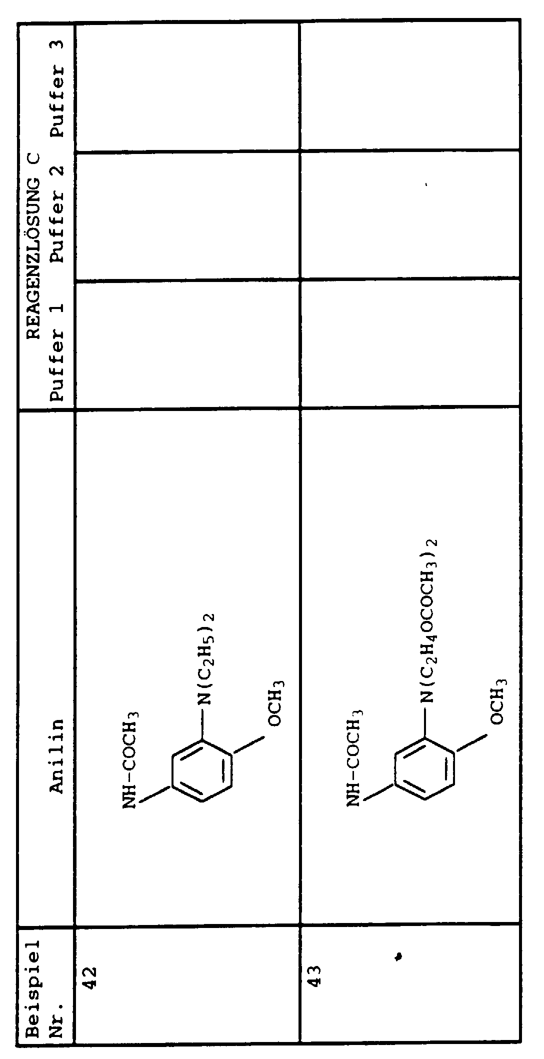

Weitere günstige Diazoniumverbindungen sind z.B. die im folgenden aufgeführten Stoffe

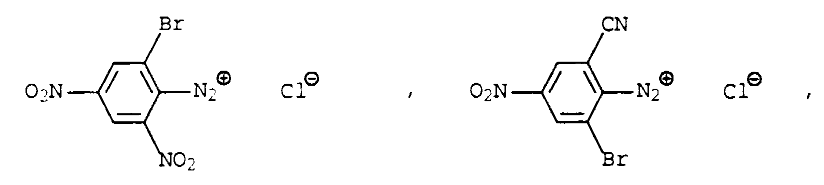

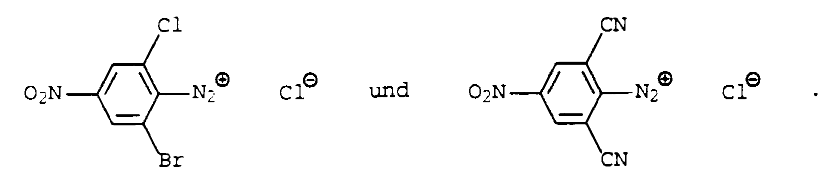

Sie ergeben bei Kupplung mit den als Markierungsmittel verwendeten Anilinen der Formel 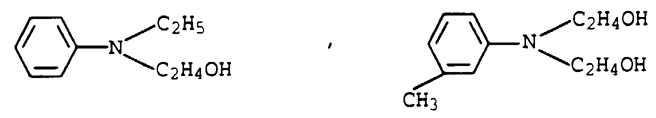 und 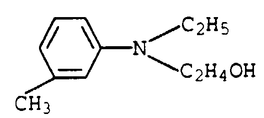 jeweils eine blaue Farbreaktion.

## Patentansprüche

1. Verwendung von Anilinen der Formel I 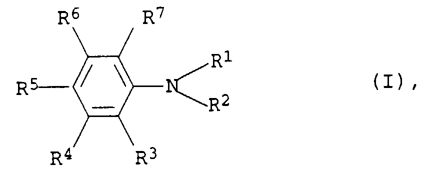 in der
R¹ und R² unabhängig voneinander jeweils Wasserstoff, C₁-C₁₈-Alkyl, das gegebenenfalls substituiert ist und durch 1 bis 3 Sauerstoffatome in Etherfunktion oder 1 bis 3 C₁-C₄-Alkyliminogruppen unterbrochen sein kann, C₃-C₁₈-Alkenyl, gegebenenfalls substituiertes Phenyl oder R¹ und R² zusammen mit dem sie verbindenden Stickstoffatom einen 5- oder 6-gliedrigen gesättigten heterocyclischen Rest, der ein weiteres Heteroatom aufweisen kann, oder R¹ auch einen Rest der Formel OL¹ oder NL¹L², worin L¹ und L² unabhängig voneinander jeweils für Wasserstoff, gegebenenfalls substituiertes C₁-C₁₈-Alkyl, C₃-C₁₈-Alkenyl oder gegebenenfalls substituiertes Phenyl stehen,
R³ und R⁷ unabhängig voneinander jeweils Wasserstoff, gegebenenfalls substituiertes C₁-C₁₈-Alkyl, C₂-C₁₈-Alkenyl, Cyano, Nitro, Formyl, gegebenenfalls substituiertes C₂-C₄-Alkanoyl, Formylamino, gegebenenfalls substituiertes C₂-C₄-Alkanoylamino, Benzoylamino oder einen Rest der Formel OL¹, CH₂COOL¹, NL¹L², SL¹ oder SO₂NL¹L², worin L¹ und L² jeweils die obengenannte Bedeutung besitzen, oder zusammen mit R² C₂-C₃-Alkylen, das gegebenenfalls ein bis dreifach durch Methyl substituiert ist, oder gegebenenfalls durch Phenyl substituierten C₂-C₃-Alkenylen,
R⁴ Wasserstoff, gegebenenfalls substituiertes C₁-C₁₈-Alkyl, C₂-C₁₈-Alkenyl, Nitro oder einen Rest der Formel OL¹, NL¹L², COOL¹ oder SO₂NL¹L², worin L¹ und L² jeweils die obengenannte Bedeutung besitzen, und
R⁵ und R⁶ unabhängig voneinander jeweils Wasserstoff, gegebenenfalls substituiertes C₁-C₁₈-Alkyl, C₂-C₁₈-Alkenyl, gegebenenfalls substituiertes Phenyl, Nitro, Formylamino, gegebenenfalls substituiertes C₂-C₄-Alkanoylamino, Benzoylamino oder einen Rest der Formel OL¹, NL¹L², SO₂NL¹L², SO₂L³, COL¹ oder COOL¹, worin L¹ und L² jeweils die obengenannte Bedeutung besitzen und L³ für gegebenenfalls substituiertes C₁-C₁₈-Alkyl, C₃-C₁₈-Alkenyl oder gegebenenfalls substituiertes Phenyl steht, bedeuten,
mit der Maßgabe, daß mindestens einer der Reste R³, R⁵ oder R⁷ Wasserstoff bedeutet,
zum Markieren von Mineralölen, wobei die Aniline der Formel I bei ihrem Nachweis als Markierungsstoff mit einem Diazoniumsalz unter Bildung eines Azofarbstoffs gekuppelt werden.

2. Verwendung von Anilinen gemäß Anspruch 1, dadurch gekennzeichnet, daß
R¹ und R² unabhängig voneinander jeweils Wasserstoff, C₁-C₁₅-Alkyl, das gegebenenfalls substituiert ist und durch 1 bis 3 Sauerstoffatome in Etherfunktion oder 1 bis 3 C₁-C₄-Alkyliminogruppen unterbrochen sein kann, Allyl, gegebenenfalls substituiertes Phenyl oder R¹ und R² zusammen mit dem sie verbindenden Stickstoffatom einen 5- oder 6-gliedrigen gesättigten heterocyclischen Rest, der ein weiteres Heteroatom aufweisen kann,
R³ und R⁷ unabhängig voneinander jeweils Wasserstoff, C₁-C₄-Alkoxy oder zusammen mit R² 1,3-Propylen, das gegebenenfalls ein- bis dreifach durch Methyl substituiert ist,
R⁴ und R⁵ jeweils Wasserstoff und
R⁶ Wasserstoff, C₁-C₄-Alkyl, C₁-C₄-Alkoxy, C₁-C₄-Alkanoylamino oder Benzoylamino bedeuten.

3. Verwendung von Anilinen gemäß Anspruch 1, dadurch gekennzeichnet, daß R¹ und R² jeweils Wasserstoff oder C₁-C₄-Alkyl oder mindestens einer der beiden Reste R¹ und R² C₂-C₁₅-Alkyl, das durch Hydroxy substituiert ist und durch 1 bis 3 Sauerstoffatome in Etherfunktion oder 1 bis 3 C₁-C₄-Alkyliminogruppen unterbrochen sein kann, und R⁶ C₁-C₄-Alkyl bedeuten.

4. Verwendung von Anilinen gemäß Anspruch 1, wobei man ihre Anwesenheit nachweist, indem man das Anilin der Formel I durch Behandeln des Mineralöls mittels eines wäßrigen Mediums extrahiert und in der wäßrigen Phase, gegebenenfalls in Gegenwart eines Puffers, mit einem Diazoniumsalz, das sich von einem Amin aus der Aminoanthrachinon-, Aminonaphthalin-, Anilin-, Aminothiophen-, Aminothiazol- oder Aminobenzisothiazolreihe ableitet, unter Bildung eines Azofarbstoffs kuppelt.

5. Verwendung von Anilinen gemäß Anspruch 1, wobei man ihre Anwesenheit nachweist, indem man das Anilin der Formel I durch Behandeln des Mineralöls mit einer sauren wäßrigen Lösung des Diazoniumsalzes extrahiert und anschließend, gegebenenfalls in Gegenwart eines Puffers, mit dem Diazoniumsalz unter Bildung eines Azofarbstoffs kuppelt.

6. Verwendung von Anilinen gemäß Anspruch 1, wobei man ihre Anwesenheit nachweist, indem man das Anilin der Formel 1 durch Behandeln des Mineralöls mittels eines wäßrigen Mediums extrahiert und in der wäßrigen Phase, gegebenenfalls in Gegenwart eines Puffers, mit dem Diazoniumsalz, das sich in festem Aggregatzustand auf einem Träger befindet, unter Bildung eines Azofarbstoffs kuppelt.

## Claims

1. Use of an aniline of the formula I 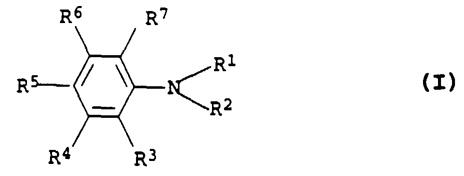 where
R¹ and R² independently of one another are each hydrogen, C₁-C₁₈-alkyl which is unsubstituted or substituted and may be interrupted by from 1 to 3 oxygen atoms in an ether function or from 1 to 3 C₁-C₄-alkylimino groups, or C₃-C₁₈-alkenyl or unsubstituted or substituted phenyl, or R¹ and R², together with the nitrogen atom which links them, may be a 5-membered or 6-membered saturated heterocyclic radical which may have a further hetero atom, or R¹ may furthermore be a radical of the formula OL¹ or NL¹L², where L¹ and L² independently of one another are each hydrogen, unsubstituted or substituted C₁-C₁₈-alkyl, C₃-C₁₈-alkenyl or unsubstituted or substituted phenyl,
R³ and R⁷ independently of one another are each hydrogen, unsubstituted or substituted C₁-C₁₈-alkyl, C₂-C₁₈-alkenyl, cyano, nitro, formyl, unsubstituted or substituted C₂-C₄-alkanoyl, formylamino, unsubstituted or substituted C₂-C₄-alkanoylamino, benzoylamino or a radical of the formula OL¹, CH₂COOL₁, NL¹L², SL¹ or SO₂NL¹L², where L¹ and L² each have the abovementioned meanings, or together with R² are C₂- or C₃-alkylene which is unsubstituted or monosubstituted to trisubstituted by methyl, or are unsubstituted or phenyl-substituted C₂- or C₃-alkenylene,
R⁴ is hydrogen, unsubstituted or substituted C₁-C₁₈-alkyl, C₂-C₁₈-alkenyl, nitro or a radical of the formula OL¹, NL¹L², COOL¹ or SO₂L¹L², where L¹ and L² each have the abovementioned meanings, and
R⁵ and R⁶ independently of one another are each hydrogen, unsubstituted or substituted C₁-C₁₈-alkyl, C₂-C₁₈-alkenyl, unsubstituted or substituted phenyl, nitro, formylamino, unsubstituted or substituted C₂-C₄-alkanoylamino, benzoylamino or a radical of the formula OL¹, NL¹L², SO₂NL¹L², SO₂L³, COL¹ or COOL¹, where L¹ and L² each have the abovementioned meanings and L³ is unsubstituted or substituted C₁-C₁₈-alkyl, C₃-C₁₈-alkenyl or unsubstituted or substituted phenyl,
with the proviso that at least one of the radicals R³, R⁵ or R⁷ is hydrogen,
for marking mineral oils, where the anilines of the formula I are, for their detection as the marker, coupled with a diazonium salt, with formation of an azo dye.

2. Use of an aniline as claimed in claim 1, wherein
R¹ and R² independently of one another are each hydrogen, C₁-C₁₅-alkyl which is unsubstituted or substituted and may be interrupted by from 1 to 3 oxygen atoms in an ether function or from 1 to 3 C₁-C₄-alkylimino groups, or allyl or unsubstituted or substituted phenyl, or R¹ and R² together with the nitrogen atom linking them are a 5-membered or 6-membered saturated heterocyclic radical which may have a further hetero atom,
R³ and R⁷ independently of one another are each hydrogen, or C₁-C₄-alkoxy or, together with R², are 1,3-propylene which is unsubstituted or monosubstituted to trisubstituted by methyl,
R⁴ and R⁵ are each hydrogen and
R⁶ is hydrogen, C₁-C₄-alkyl, C₁-C₄-alkoxy, C₁-C₄-alkanoylamino or benzoylamino.

3. Use of an aniline as claimed in claim 1, wherein R¹ and R² are each hydrogen or C₁-C₄-alkyl or at least one of the two radicals R¹ and R² is C₂-C₁₅-alkyl which is substituted by hydroxyl and may be interrupted by from 1 to 3 oxygen atoms in an ether function or from 1 to 3 C₁-C₄-alkylimino groups, and R⁶ is C₁-C₄-alkyl.

4. Use of an aniline as claimed in claim 1, wherein the presence of the aniline is detected by extracting the aniline of the formula I by treating the mineral oil with an acidic medium and coupling the aniline in the aqueous phase, in the presence or absence of a buffer, with a diazonium salt derived from an amine from the aminoanthraquinone, aminonaphthalene, aniline, aminothiophene, aminothiazole or aminobenzothiazole series, with formation of an azo dye.

5. Use of an aniline as claimed in claim 1, wherein the presence of the aniline is detected by extracting the aniline of the formula I by treating the mineral oil with an acidic aqueous solution of the diazonium salt, and then coupling the aniline, in the presence or absence of a buffer, with the diazonium salt with formation of an azo dye.

6. Use of an aniline as claimed in claim 1, wherein the presence of the aniline is detected by extracting the aniline of the formula I by treating the mineral oil with an aqueous medium, and coupling the aniline in the aqueous phase, in the presence or absence of a buffer, with the diazonium salt which is present in the solid state on a substrate, with formation of an azo dye.

## Revendications

1. Utilisation d'anilines de formule I 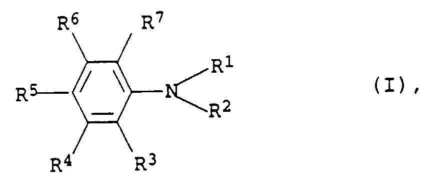 dans laquelle
R¹ et R² représentent chacun, indépendamment l'un de l'autre, un atome d'hydrogène, un reste alkyle en C₁-C₁₈ qui est éventuellement substitué et peut être interrompu par 1 à 3 atomes d'oxygène en fonction éther ou 1 à 3 groupements alkylimino en C₁-C₄, un reste alcényle en C₃-C₁₈, phényle éventuellement substitué, ou bien R¹ et R² représentent ensemble, avec l'atome d'azote qui les relie, un reste hétéroxyclique saturé à 5 ou 6 chaînons qui peut comporter un autre hétéroatome, ou bien R¹ peut aussi représenter un reste de formule OL¹ ou NL¹L², dans laquelle L¹ et L² sont mis chacun, indépendamment l'un de l'autre, pour un atome d'hydrogène, un reste alkyle en C₁-C₁₈ éventuellement substitué, un reste alcényle en C₃-C₁₈ ou phényle éventuellement substitué,
R³ et R⁷ représentent chacun, indépendamment l'un de l'autre, un atome d'hydrogène, un reste alkyle en C₁-C₁₈ éventuellement substitué, un reste alcényle en C₂-C₁₈, cyano, nitro, formyle, alcanoyle en C₂-C₄ éventuellement substitué, formylamino, alcanoylamino en C₂-C₄ éventuellement substitué, benzoylamino ou un reste de formule OL¹, CH₂COOL¹, NL¹L², SL¹ ou SO₂NL¹L² dans laquelle L¹ et L² ont chacun la signification donnée ci-dessus, ou bien ils représentent ensemble, avec R², un reste alkylène en C₂-C₃, qui est éventuellement substitué une à trois fois par des groupements méthyle, ou un reste alcénylène en C₂-C₃ éventuellement substitué par un groupement phényle,
R⁴ représente un atome d'hydrogène, un reste alkyle en C₁-C₁₈ éventuellement substitué, alcényle en C₂-C₁₈, nitro ou un reste de formule OL¹, NL¹L², COOL¹ ou SO₂NL¹L², dans laquelle L¹ et L² ont chacun la signification donnée ci-dessus, et
R⁵ et R⁶ représentent chacun, indépendamment l'un de l'autre, un atome d'hydrogène, un reste alkyle en C₁-C₁₈ éventuellement substitué, alcényle en C₂-C₁₈, phényle éventuellement substitué, nitro, formylamino, alcanoylamino en C₂-C₄ éventuellement substitué, benzoylamino ou un reste de formule OL¹, NL¹L², SO₂NL¹L², SO₂L³, COL¹ ou COOL¹, dans laquelle L¹ et L² ont chacun la signification donnée ci-dessus et L³ est mis pour un reste alkyle en C₁-C₁₈ éventuellement substitué, alcényle en C₃-C₁₈ ou phényle éventuellement substitué,
étant spécifié que l'un au moins des restes R³, R⁵ ou R⁷ est un atome d'hydrogène,
pour le marquage d'huiles minérales, les anilines de formule I étant copulées, lors de leur décèlement comme substance de marquage, avec un sel de diazonium en formant un colorant azoïque.

2. Utilisation d'anilines selon la revendication 1, caractérisées en ce que
R¹ et R² représentent chacun, indépendamment l'un de l'autre, un atome d'hydrogène, un reste alkyle en C₁-C₁₅, qui est éventuellement substitué et peut être interrompu par 1 à 3 atomes d'oxygène en fonction éther ou 1 à 3 groupements alkylimino en C₁-C₄, un reste allyle, phényle éventuellement substitué, ou bien R¹ et R² représentent ensemble, avec l'atome d'azote qui les relie, un reste hétérocyclique saturé à 5 ou 6 chaînons qui peut comporter un autre hétéroatome,
R³ et R⁷ représentent chacun, indépendamment l'un de l'autre, un atome d'hydrogène ou un reste alcoxy en C₁-C₄, ou bien ils représentent ensemble, avec R², un reste 1,3-propylène qui est éventuellement substitué une à trois fois par des groupements méthyle,
R⁴ et R⁵ représentent chacun un atome d'hydrogène et
R⁶ représente un atome d'hydrogène ou un reste alkyle en C₁-C₄, alcoxy en C₁-C₄, alcanoylamino en C₁-C₄ ou benzoylamino.

3. Utilisation d'anilines selon la revendication 1, caractérisées en ce que R¹ et R² représentent chacun un atome d'hydrogène ou un reste alkyle en C₁-C₄, ou bien l'un au moins des deux restes R¹ et R² représente un reste alkyle en C₂-C₁₅ qui est substitué par un groupement hydroxy et peut être interrompu par 1 à 3 atomes d'oxygène en fonction éther ou 1 à 3 groupements alkylimino en C₁-C₄, et R⁶ représente un reste alkyle en C₁-C₄.

4. Utilisation d'anilines selon la revendication 1, dans laquelle on décèle la présence de celles-ci en extrayant l'aniline de formule I par traitement de l'huile minérale au moyen d'un milieu aqueux et en la copulant dans la phase aqueuse, éventuellement en présence d'un tampon, avec un sel de diazonium qui dérive d'une amine de la série des aminoanthraquinones, des aminonaphtalènes, des anilines, des aminothiophènes, des aminothiazoles ou des aminobenzisothiazoles, avec formation d'un colorant azoïque.

5. Utilisation d'anilines selon la revendication 1, dans laquelle on décèle la présence de celles-ci en extrayant l'aniline de formule I par traitement de l'huile minérale par une solution aqueuse acide du sel de diazonium, puis en la copulant, éventuellement en présence d'un tampon, avec le sel de diazonium pour former un colorant azoïque.

6. Utilisation d'anilines selon la revendication 1, dans laquelle on décèle la présence de celles-ci en extrayant l'aniline de formule I par traitement de l'huile minérale au moyen d'un milieu aqueux et en la copulant dans la phase aqueuse, éventuellement en présence d'un tampon, avec le sel de diazonium qui se trouve à l'état physique solide sur un support, avec formation d'un colorant azoïque.
